# EUROPEAN PATENT SPECIFICATION

(11) **EP 4 177 766 B1**
(45) Date of publication and mention of the grant of the patent: **22.10.2025**
(21) Application number: 20943751.6
(22) Date of filing: 03.07.2020
(51) Int. Cl.: G16B 50/20, G06F 16/00, G16B 40/20, G16H 70/40

(54) **INFORMATION PROCESSING PROGRAM, INFORMATION PROCESSING METHOD, AND INFORMATION PROCESSING DEVICE**
INFORMATIONSVERARBEITUNGSPROGRAMM, INFORMATIONSVERARBEITUNGSVERFAHREN UND INFORMATIONSVERARBEITUNGSVORRICHTUNG
PROGRAMME DE TRAITEMENT D'INFORMATIONS, PROCÉDÉ DE TRAITEMENT D'INFORMATIONS ET DISPOSITIF DE TRAITEMENT D'INFORMATIONS

(43) Date of publication of application: 10.05.2023
(73) Proprietor: FUJITSU LIMITED, Kawasaki-shi, Kanagawa 211-8588 (JP)
(72) Inventor: KATAOKA, Masahiro, Kawasaki-shi, Kanagawa 211-8588 (JP); ONOUE, Satoshi, Kawasaki-shi, Kanagawa 211-8588 (JP); SUGIYAMA, Kokichi, Kawasaki-shi, Kanagawa 211-8588 (JP)
(74) Representative: Hoffmann Eitle
(86) International application number: PCT/JP2020/026214
(87) International publication number: WO 2022/003956

(56) References cited:
- JP-A- 2018 045 657
- JP-A- 2018 060 463
- JP-A- 2020 009 203
- JP-A- 2020 035 134
- KE YU ET AL: "Semi-Supervised Hierarchical Drug Embedding in Hyperbolic Space", ARXIV.ORG, CORNELL UNIVERSITY LIBRARY, 201 OLIN LIBRARY CORNELL UNIVERSITY ITHACA, NY 14853, 2 June 2020 (2020-06-02), XP081689807
- INES CHAMI ET AL: "Hyperbolic Graph Convolutional Neural Networks", ARXIV.ORG, CORNELL UNIVERSITY LIBRARY, 201 OLIN LIBRARY CORNELL UNIVERSITY ITHACA, NY 14853, 28 October 2019 (2019-10-28), XP081522246
- SINGH APOORVA VIKRAM ET AL: "Towards Better Drug Repositioning Using Joint Learning", 2019 IEEE 16TH INDIA COUNCIL INTERNATIONAL CONFERENCE (INDICON), IEEE, 13 December 2019 (2019-12-13), pages 1 - 4, XP033738262, DOI: 10.1109/INDICON47234.2019.9029004
- DAI JIAN DAIJ@COMP NUS EDU SG ET AL: "Fine-grained Concept Linking using Neural Networks in Healthcare", PROCEEDINGS OF THE 27TH ANNUAL INTERNATIONAL CONFERENCE ON MOBILE COMPUTING AND NETWORKING, ACM, NEW YORK, NY, USA, 27 May 2018 (2018-05-27), pages 51 - 66, XP058753826, ISBN: 978-1-4503-8342-4, DOI: 10.1145/3183713.3196907
- XIANG ZHAO ET AL: "A partition-based approach to structure similarity search", PROCEEDINGS OF THE VLDB ENDOWMENT; [ACM DIGITAL LIBRARY], ASSOC. OF COMPUTING MACHINERY, NEW YORK, NY, vol. 7, no. 3, 1 November 2013 (2013-11-01), pages 169 - 180, XP058064014, ISSN: 2150-8097, DOI: 10.14778/2732232.2732236

## Description

### FIELD

The present invention relates to an information processing program and the like.

### BACKGROUND

There are substances with a molecular weight of more than 1000, such as starch, cellulose, or natural rubber, and such substances are also called high-molecular compounds. A structure of the high-molecular compound (compound) is a high-order structure including primary structures of a plurality of groups (functional group). Furthermore, structures of cancer cells or human body cells are high-order structures including primary structures of a plurality of proteins.

Here, in a case where a new material or a new drug is developed, it is requested to search for a high-molecular compound or a cell that has been already confirmed and to evaluate a similarity. For example, as a technique for evaluating the similarity of the compounds or cells, there is a technique for assigning vectors to description formulas of the compounds and the proteins and evaluating the similarity.
Ke Yu et al., "Semi-Supervised Hierarchical Drug Embedding in Hyperbolic Space",ARXIV.ORG, CORNELL UNIVERSITY LIBRARY, 201 OLIN LIBRARY CORNELL UNIVERSITY ITHACA, NY 14853, 2 June 2020 discloses the use of a Variational Auto-Encoder framework to encode the chemical structures of molecules and the use of knowledge-based drug-drug similarity to induce the clustering of drugs in hyperbolic space.

### SUMMARY

### TECHNICAL PROBLEM

In the related art described above, a vector that has been determined in advance according to characteristics of a descriptor is assigned to the descriptor of the group of the compound or the cellular protein. Therefore, there is a case where the vectors of the groups and the proteins are deviated, and it is not possible to assign an appropriate vector. Furthermore, in the related art, it is not possible to perform similarity search with various granularities of a primary structure and a high-order structure of each of the group of the compound and the cellular protein.

In one aspect, an object of the present invention is to provide an information processing program, an information processing method, and an information processing device that can accurately perform similarity search with various granularities of a primary structure and a high-order structure of each of a group of a compound and a cellular protein.

### SOLUTION TO PROBLEM

In a first option, a computer is caused to execute following processing. A computer calculates vectors of a plurality of pieces of basic information by performing Poincare Embeddings on the plurality of pieces of basic information, based on a common concept table that classifies the plurality of pieces of space-specific basic information defined in a plurality of spaces with a common concept. The computer calculates a vector of structural information with a granularity larger than the basic information, based on the vectors of the plurality of pieces of basic information. The computer generates an inverted index that defines a relationship between a position of the basic information in a file corresponding to the same space and the vector of the basic information and a relationship between a position of the structural information in the file and the vector of the structural information.

### Summary of the invention

The invention is defined by the independent claims. Preferred embodiments are defined in the dependent claims.

### ADVANTAGEOUS EFFECTS OF INVENTION

It is possible to accurately execute similarity search with various granularities of a primary structure and a high-order structure of each of groups of compounds and cellular proteins.

### BRIEF DESCRIPTION OF DRAWINGS

FIG. 1 is a diagram (1) for explaining processing of an information processing device according to a first embodiment.
FIG. 2 is a diagram (2) for explaining the processing of the information processing device according to the first embodiment.
FIG. 3 is a diagram (3) for explaining the processing of the information processing device according to the first embodiment.
FIG. 4 is a diagram (4) for explaining the processing of the information processing device according to the first embodiment.
FIG. 5 is a diagram (5) for explaining the processing of the information processing device according to the first embodiment.
FIG. 6 is a functional block diagram illustrating a configuration of the information processing device according to the first embodiment.
FIG. 7 is a diagram illustrating an example of a data structure of a base file.
FIG. 8 is a diagram illustrating an example of a data structure of a protein dictionary.
FIG. 9 is a diagram illustrating a relationship between amino acids, bases, and codons.
FIG. 10 is a diagram illustrating an example of a data structure of a chemical structural formula file.
FIG. 11 is a diagram illustrating an example of a data structure of a group dictionary.
FIG. 12 is a diagram illustrating an example of a group primary structure.
FIG. 13 is a diagram illustrating an example of a data structure of a common concept table.
FIG. 14 is a diagram illustrating an example of a data structure of a conversion table.
FIG. 15 is a diagram illustrating an example of a data structure of a dictionary table.
FIG. 16 is a diagram illustrating an example of a data structure of a primary structure dictionary.
FIG. 17 is a diagram illustrating an example of a data structure of a high-order structure dictionary.
FIG. 18 is a diagram illustrating an example of a data structure of a group primary dictionary.
FIG. 19 is a diagram illustrating an example of a data structure of a word dictionary.
FIG. 20 is a diagram illustrating an example of a data structure of a vector table.
FIG. 21 is a diagram illustrating an example of a data structure of a protein vector table.
FIG. 22 is a diagram illustrating an example of a data structure of a primary structure vector table.
FIG. 23 is a diagram illustrating an example of a data structure of a high-order structure vector table.
FIG. 24 is a diagram illustrating an example of a data structure of a group vector table.
FIG. 25 is a diagram illustrating an example of a data structure of a group primary structure vector table.
FIG. 26 is a diagram illustrating an example of a data structure of a name vector table.
FIG. 27 is a diagram illustrating an example of a data structure of an inverted index table.
FIG. 28 is a diagram illustrating an example of a data structure of a protein inverted index.
FIG. 29 is a diagram illustrating an example of a data structure of a primary structure inverted index.
FIG. 30 is a diagram illustrating an example of a data structure of a high-order structure inverted index.
FIG. 31 is a diagram illustrating an example of a data structure of a group inverted index.
FIG. 32 is a diagram illustrating an example of a data structure of a group primary structure inverted index.
FIG. 33 is a diagram illustrating an example of a data structure of a name inverted index.
FIG. 34 is a flowchart (1) illustrating a processing procedure of the information processing device according to the first embodiment.
FIG. 35 is a flowchart (2) illustrating the processing procedure of the information processing device according to the first embodiment.
FIG. 36 is a diagram for explaining another processing of a calculation unit.
FIG. 37 is a functional block diagram illustrating a configuration of an information processing device according to a second embodiment.
FIG. 38 is a diagram illustrating an example of a data structure of teacher data.
FIG. 39 is a diagram illustrating an example of a hardware configuration of a computer that implements functions similar to those of the information processing device according to the embodiments.

### DESCRIPTION OF EMBODIMENTS

Hereinafter, embodiments of an information processing program, an information processing method, and an information processing device disclosed in the present application will be described in detail with reference to the drawings. Note that the embodiments do not limit the present invention.

### [First Embodiment]

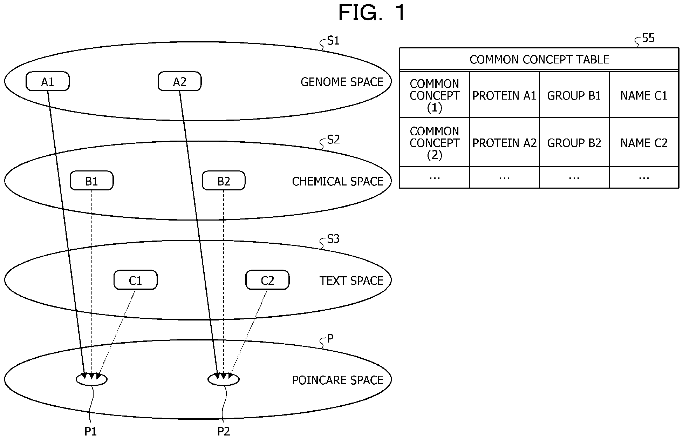

An example of processing of an information processing device according to a first embodiment will be described. FIGs. 1 to 5 are diagrams for explaining the processing of the information processing device according to the first embodiment. The information processing device calculates vectors of a plurality of pieces of basic information by performing Poincare Embeddings on the plurality of pieces of basic information based on a common concept table 55 that classifies the plurality of pieces of space-specific basic information defined in a plurality of spaces with a common concept.

In the example illustrated in FIG. 1, description will be made while using a genome space S1, a chemical space S2, and a text space S3 as the plurality of spaces. The genome space S1 is a space including information regarding a protein (may be referred to as "a basic structure of the protein), and a primary structure and a high-order structure of the protein. For example, a "base file" includes the information in the genome space S1. The base file is information in which a plurality of base symbols A, G, C, and T (or U) is arranged and a codon that is a combination of three bases corresponds to a predetermined amino acid. Furthermore, a combination of a plurality of consecutive amino acids corresponds to a predetermined protein (i.e., a basic structure of the predetermined protein), and a combination of a plurality of proteins corresponds to a primary structure. Moreover, a combination of the plurality of primary structures is a high-order structure.

The chemical space S2 is a space including information regarding a compound group (functional group) and a primary structure in which a plurality of groups is connected. For example, a "chemical structural formula file" includes the information in the chemical space S2. The chemical structural formula file is information including a rational formula of a plurality of groups, and a rational formula of primary structures is obtained by combining the rational formulas of the groups in minimum units.

The text space S3 is a space including a name of a protein (may be referred to as "a name of a basic structure of the protein"), a name of a primary structure of the proteins, a name of a high-order structure of the proteins, a name of a group (functional group), a name of a primary structure of the groups, and a name of a high-order structure of the groups. For example, a "document file" includes information in the text space S3. The document file is a text file including character strings regarding the protein, the primary structure of the proteins, the group of the compounds, and the primary structure of the groups. In the following description, the primary structure of the proteins is referred to as a "primary structure", and the primary structure of the groups is referred to as a "group primary structure".

For example, the genome space S1 corresponds to a first space. The chemical space S2 corresponds to a second space. The text space S3 corresponds to a third space. The protein in the genome space S1, the group in the chemical space S2, and the name in the text space S3 correspond to the basic information (first basic information, second basic information, and third basic information). The primary structure and the high-order structure in the genome space S1 and the group primary structure and the group high-order structure in the chemical space S2 correspond to structural information (first structural information).

In the common concept table 55, the basic information of the genome space S1, the chemical space S2, and the text space S3 that is a common concept is defined. In the example illustrated in FIG. 1, it is defined that a "protein A1" in the genome space S1, a "group B1" in the chemical space S2, and a "name C1" in the text space S3 are the same common concept (1). It is defined that a "protein A2" in the genome space S1, a "group B2" in the chemical space S2, and a "name C2" in the text space S3 are the same common concept (2).

Here, the information processing device calculates a vector of the basic information by embedding the basic information of the genome space S1, the chemical space S2, and the text space S3 in a Poincare space P. Processing for embedding the information in the Poincare space P and calculating the vector is a technique called Poincare Embeddings. For Poincare Embeddings, for example, the technique described in Non-Patent Document "Valentin Khrulkov et al. "Hyperbolic Image Embeddings" Cornell University, April 3, 2019", and the like may be used.

In Poincare embeddings, a vector is assigned according to an embedded position in the Poincare space P, and the more similar information is, the closer the positions where information is embedded are. Therefore, since respective pieces of the basic information classified into the same common concept are embedded at positions close to each other in the Poincare space P, similar vectors are assigned.

For example, the "protein A1" in the genome space S1, the "group B1" in the chemical space S2, and the "name C1" in the text space S3 are embedded into a space P1 in the Poincare space P, and a vector according to the space P1 is assigned.

The "protein A2" in the genome space S1, the "group B2" in the chemical space S2, and the "name C2" in the text space S3 are embedded into a space P2 in the Poincare space P, and a vector according to the space P2 is assigned.

The information processing device calculates each of the vector of each protein in the genome space S1, the vector of each group in the chemical space S2, and the vector of each name in the text space S3 using the common concept table 55.

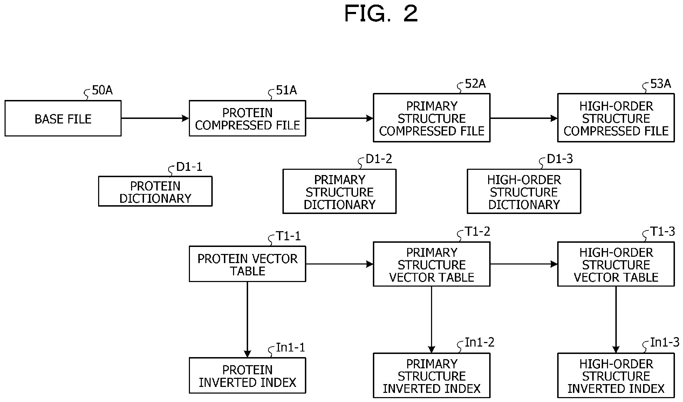

The description proceeds to FIG. 2. The information processing device converts a base file 50A into a protein compressed file 51A using a protein dictionary D1-1. The protein dictionary D1-1 is dictionary data that defines a relationship between information regarding the base file 50A and a compression code of a protein.

The information processing device generates a protein vector table T1-1 in which the protein (compression code of protein) is associated with a vector of the protein. The vector of the protein is calculated through Poincare Embeddings described above. The information processing device generates a protein inverted index In1-1 that defines a relationship between the vector of the protein and a position of the protein in the protein compressed file 51A.

Subsequently, the information processing device converts the protein compressed file 51A into a primary structure compressed file 52A using a primary structure dictionary D1-2. The primary structure dictionary D1-2 is dictionary data that defines a relationship between a sequence of the compression codes of the proteins and a compression code of the primary structure.

The information processing device specifies the compression code of the protein included in the primary structure (compression code of primary structure) based on the primary structure dictionary D1-2, and acquires a vector corresponding to the specified compression code of the protein from the protein vector table T1-1. The information processing device calculates a vector of the primary structure by adding the vectors of the proteins included in the primary structure. The information processing device calculates the vector of each primary structure by executing the processing described above for each primary structure. A primary structure vector table T1-2 in which the primary structure (compression code of primary structure) and the vector of the primary structure are associated is generated.

The information processing device generates a primary structure inverted index In1-2 that defines a relationship between the vector of the primary structure and a position of the primary structure in the primary structure compressed file 52A.

Subsequently, the information processing device compresses the primary structure compressed file 52A into a high-order structure compressed file 53A using a high-order structure dictionary D1-3. The high-order structure dictionary D1-3 is dictionary data that defines a relationship between a sequence of the compression codes of the primary structures and a compression code of the high-order structure.

The information processing device specifies a compression code of the primary structure included in the high-order structure (compression code of high-order structure) based on the high-order structure dictionary D1-3 and acquires a vector corresponding to the specified compression code of the primary structure from the primary structure vector table T1-2. The information processing device calculates a vector of the high-order structure by adding the vectors of the primary structures included in the high-order structure. The information processing device calculates the vector of each high-order structure by executing the processing described above for each high-order structure. A high-order structure vector table T1-3 is generated in which the high-order structure (compression code of high-order structure) and the vector of the high-order structure are associated.

The information processing device generates a high-order structure inverted index In1-3 that defines a relationship between the vector of the high-order structure and a position of the high-order structure in the high-order structure compressed file 53A.

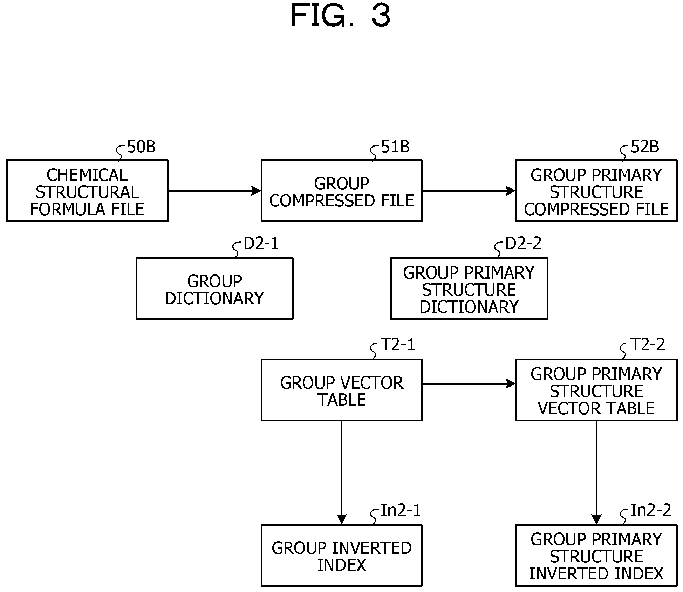

The description proceeds to FIG. 3. The information processing device converts a chemical structural formula file 50B into a group compressed file 51B using a group dictionary D2-1. The group dictionary D2-1 is dictionary data that defines a relationship between a rational formula of the chemical structural formula file 50B and a compression code of a group (functional group).

The information processing device generates a group vector table T2-1 in which a group (compression code of group) and a vector of the group are associated. The vector of the group is calculated through Poincare Embeddings described above. The information processing device generates a group inverted index In2-1 that defines a relationship between the vector of the group and a position of the group in the group compressed file 51B.

Subsequently, the information processing device converts the group compressed file 51B into a group primary structure compressed file 52B using a group primary structure dictionary D2-2. The group primary structure compressed file 52B is dictionary data that defines a relationship between a sequence of the compression codes of the groups and a compression code of the group primary structure.

The information processing device specifies a compression code of the group included in the group primary structure (compression code of group primary structure) based on the group primary structure dictionary D2-2 and acquires a vector corresponding to the specified compression code of the group from the group vector table T2-1. The information processing device calculates a vector of the group primary structure by adding the vectors of the groups included in the group primary structure. The information processing device calculates the vector of each group primary structure by executing the processing described above for each group primary structure. The information processing device generates a group primary structure vector table T2-2 in which the group primary structure (compression code of group primary structure) and the vector of the group primary structure are associated.

The information processing device generates a group primary structure inverted index In2-2 that defines a relationship between the vector of the group primary structure and a position of the primary structure in the group primary structure compressed file 52B.

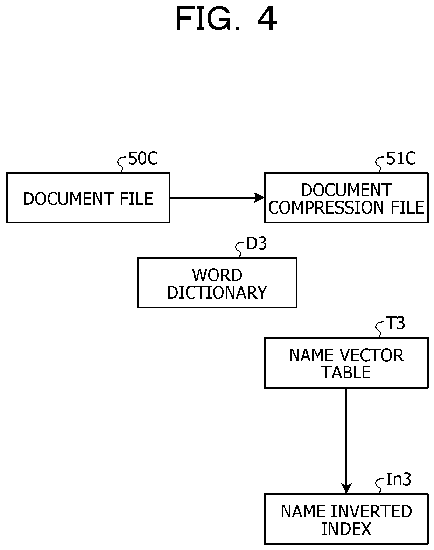

The description proceeds to FIG. 4. The information processing device converts a document file 50C into a document compression file 51C using a word dictionary D3. The word dictionary D3 is a dictionary that defines compression codes for a name of a protein, a name of a group, a name of a primary structure of the protein, a name of a group primary structure, a name of a high-order structure, and the like.

The information processing device generates a name vector table T3 in which a name (compression code of name) and a vector of the name are associated. For example, the name includes the name of the protein, the name of the group, the name of the primary structure of the protein, the name of the primary structure of the group, and the name of the high-order structure. The vector of the name is calculated through Poincare Embeddings described above. The information processing device generates a name inverted index In3 that defines a relationship between the vector of the name and a position of the name in the document compression file 51C.

By executing the processing in FIGs. 1 to 4, the information processing device generates the protein inverted index In1-1, the primary structure inverted index In1-2, and the high-order structure inverted index In1-3. The information processing device generates the protein compressed file 51A, the primary structure compressed file 52A, and the high-order structure compressed file 53A from the base file 50A. The information processing device generates the group inverted index In2-1 and the group primary structure inverted index In2-2. The information processing device generates the group compressed file 51B and the group primary structure compressed file 52B from the chemical structural formula file 50B. The information processing device generates the name inverted index In3. The information processing device generates the document compression file 51C from the document file 50C.

In the following description, the protein inverted index In1-1, the primary structure inverted index In1-2, and the high-order structure inverted index In1-3 are collectively referred to as an "inverted index In1". The group inverted index In2-1 and the group primary structure inverted index In2-2 are collectively referred to as an "inverted index In2".

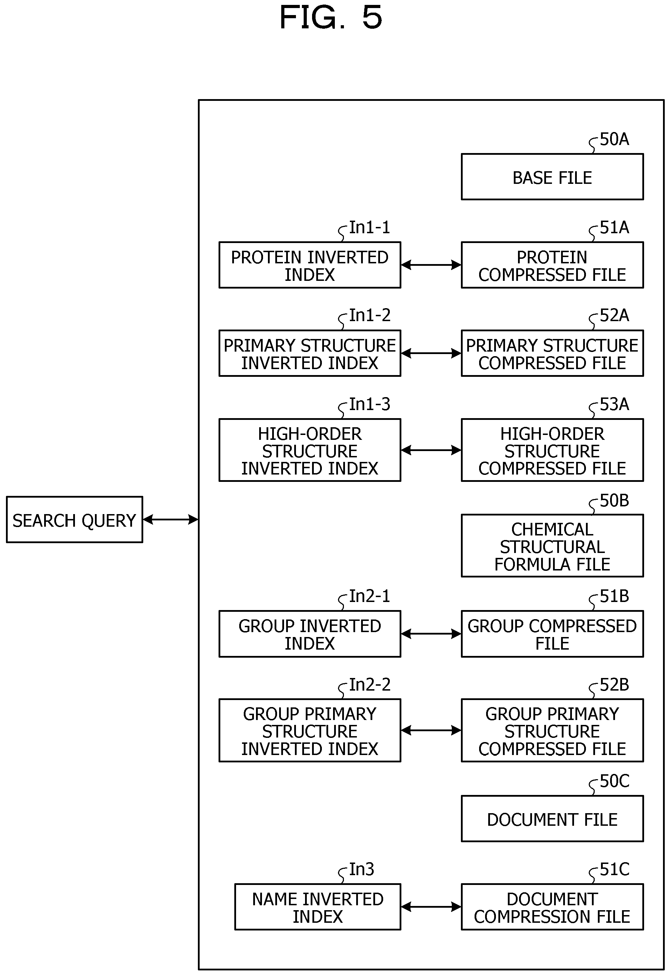

The description proceeds to FIG. 5. In a case of receiving a search query, the information processing device evaluates a similarity between a vector of the search query and vectors of the inverted indexes In1 to In3. The information processing device searches for a vector similar to the vector of the search query from the vectors of the inverted indexes In1 to In3. The vector similar to the vector of the search query is referred to as a "similar vector".

The information processing device acquires information regarding a protein, a primary structure, or a high-order structure corresponding to the similar vector, based on the similar vector and the inverted index In1, from the protein compressed file 51A, the primary structure compressed file 52A, and the high-order structure compressed file 53A. The information processing device acquires information regarding a group or a primary structure corresponding to the similar vector, based on the similar vector and the inverted index In2, from the group compressed file 51B and the group primary structure compressed file 52B. The information processing device acquires a name (or document data related to name) corresponding to the similar vector, based on the similar vector and the inverted index In3, from the document compression file 51C.

The information processing device outputs the acquired information as a search result for the search query. Since the information acquired by the information processing device is encoded, the information may be decoded using each piece of dictionary information.

As described with reference to FIGs. 1 to 5, according to the information processing device according to the first embodiment, Poincare Embeddings is performed on the information regarding the plurality of spaces based on the common concept table 55 and the vector is assigned. The information processing device generates the inverted indexes In1 to In3 in which the vector assigned to the information and a position of the information are assigned. In a case of receiving the search query, the information processing device acquires information similar to the search query from the plurality of spaces, based on the vector of the search query and the inverted indexes In1 to In3. As a result, similarity search can be accurately performed with various granularities such as high-order structures of compounds, primary structures, or proteins.

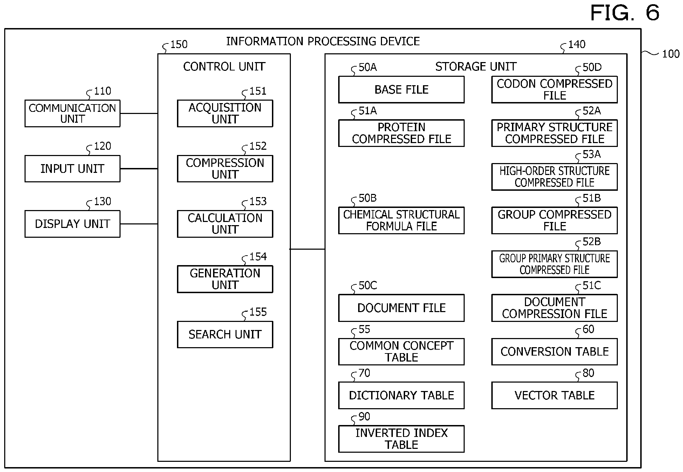

Next, an example of a configuration of the information processing device according to the first embodiment will be described. FIG. 6 is a functional block diagram illustrating the configuration of the information processing device according to the first embodiment. As illustrated in FIG. 6, an information processing device 100 according to the first embodiment includes a communication unit 110, an input unit 120, a display unit 130, a storage unit 140, and a control unit 150.

The communication unit 110 is wiredly or wirelessly connected to an external device and the like, and exchanges information with the external device and the like. For example, the communication unit 110 is implemented by a network interface card (NIC) and the like. The communication unit 110 may be connected to a network (not illustrated).

The input unit 120 is an input device that inputs various types of information to the information processing device 100. The input unit 120 corresponds to a keyboard, a mouse, a touch panel, and the like.

The display unit 130 is a display device that displays information output from the control unit 150. The display unit 130 corresponds to a liquid crystal display, an organic electro luminescence (EL) display, a touch panel, and the like.

The storage unit 140 includes the base file 50A, the protein compressed file 51A, the primary structure compressed file 52A, and the high-order structure compressed file 53A. The storage unit 140 includes the chemical structural formula file 50B, the group compressed file 51B, and the group primary structure compressed file 52B. The storage unit 140 includes the document file 50C, the document compression file 51C, the common concept table 55, a conversion table 60, a dictionary table 70, a vector table 80, and an inverted index table 90. The storage unit 140 is implemented by, for example, a semiconductor memory element such as a random access memory (RAM) or a flash memory, or a storage device such as a hard disk or an optical disk.

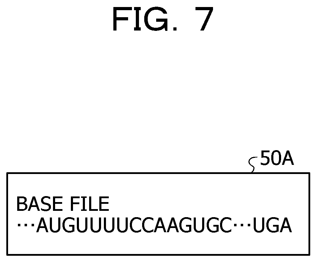

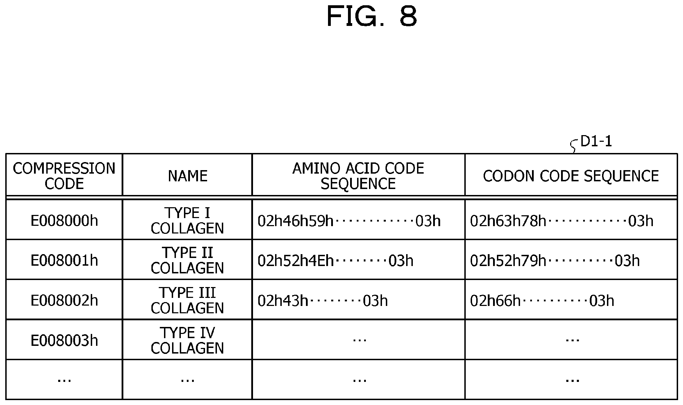

The base file 50A is a file that holds information in which a plurality of bases is arranged. FIG. 7 is a diagram illustrating an example of a data structure of a base file. There are four types of bases of a DNA or RNA that are indicated by symbols of "A", "G", "C", "T", or "U". Furthermore, FIG. 8 is a diagram illustrating an example of a structure of a protein dictionary. The protein dictionary indicates a compression code of a protein and an amino acid (or codon) sequence configuring the protein. On the other hand, three base sequences are collectively referred to as 64 types of codons and determine 20 types of amino acids. Each of the amino acids is indicated by symbols of "A" to "Y".

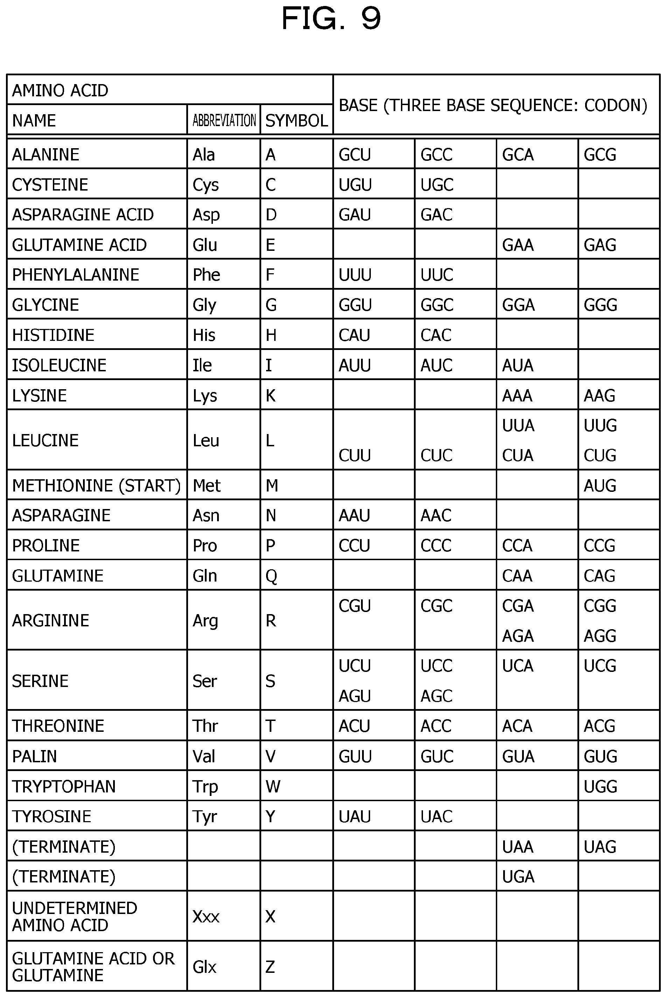

FIG. 9 is a diagram illustrating a relationship between an amino acid, a base, and a codon. As illustrated in FIG. 9, a plurality of types of codons is associated with one amino acid. Thus, when the codon is determined, the amino acid is determined. However, even when the amino acid is determined, the codon is not uniquely specified. For example, the amino acid "alanine (Ala)" is associated with codons "GCU", "GCC", "GCA", or "GCG".

A codon compressed file 50D is a file obtained by compressing a base included in the base file 50A in codon units.

The protein compressed file 51A is a file obtained by encoding a sequence of the compression codes of codons included in the codon compressed file 50D in protein units.

The primary structure compressed file 52A is a file obtained by encoding a sequence of compression codes of the proteins included in the protein compressed file 51A in primary structure units.

The high-order structure compressed file 53A is a file obtained by encoding a sequence of compression codes of the primary structures included in the primary structure compressed file 52A in high-order structure units.

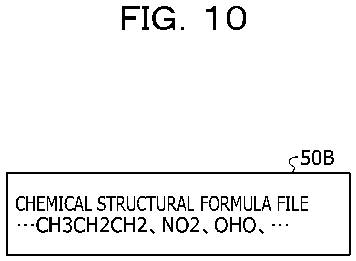

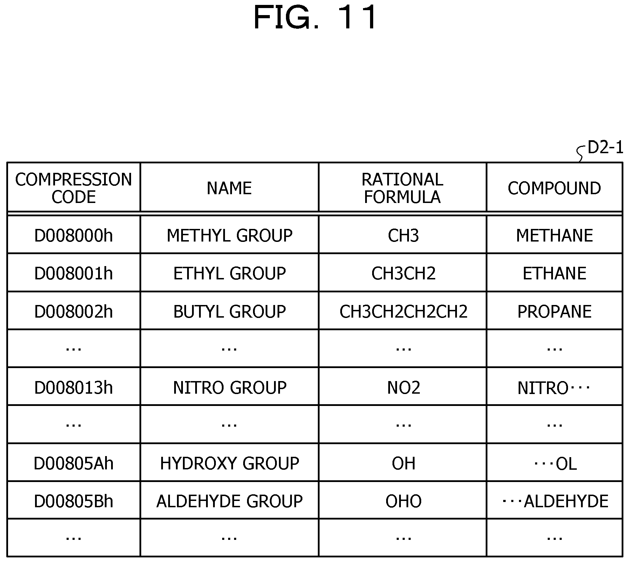

The chemical structural formula file 50B is a file that holds rational formulas (chemical structural formula) of a plurality of group primary structures. For example, the group primary structure corresponds to ranitidine, cimetidine, ranitidine, and the like. However, the group primary structure is not limited to this and may be another high-molecular compound. FIG. 10 is a diagram illustrating an example of a data structure of a chemical structural formula file. A rational formula (chemical structural formula) is a formula indicating a sequence of elements included in a compound and may be written in the SMILES notation and the like. Furthermore, FIG. 11 illustrates an example of a structure of a group dictionary. The group dictionary defines a compression code of a group and a sequence of elements included in the group as a rational formula.

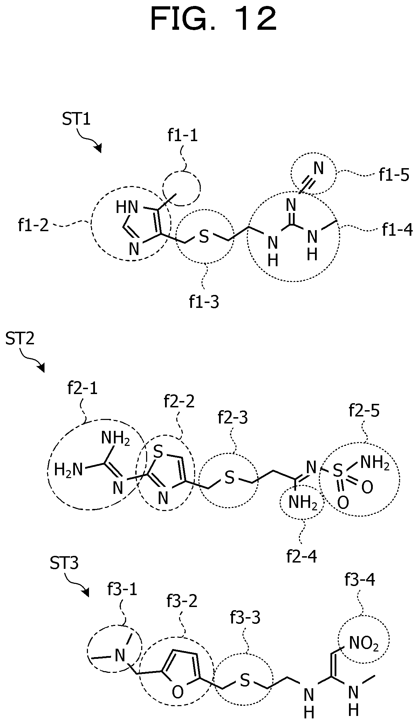

FIG. 12 is a diagram illustrating an example of a group primary structure. For example, the group primary structure includes cimetidine, famotidine, ranitidine, and the like, and these are antagonists to be bind to a histamine H₂ receptor that have similar properties. Note that the group primary structure is not limited to these antagonists and may be other compounds. A group primary structure ST1 illustrated in FIG. 12 is a group primary structure of cimetidine. The group primary structure ST1 includes a methyl group f1-1, an imidazole ring f1-2, sfild f1-3, a guanidinomethyl group f1-4, a cyano group f1-5, and the like.

A group primary structure ST2 is a group primary structure of famotidine. The group primary structure ST2 includes a guanidino group f2-1, a thiazole ring f2-2, sfide f2-3, an amino group f2-4, a sulfonamino f2-5, and the like.

A group primary structure ST3 is a group primary structure of ranitidine. The group primary structure ST3 includes a dimethylamino group f3-1, a furan ring f3-2, sfide f3-3, a nitro group f3-4, and the like.

The group compressed file 51B is a file obtained by encoding the chemical structural formula file 50B in group units.

The group primary structure compressed file 52B is a file obtained by encoding a sequence of compression codes of groups included in the group compressed file 51B in group primary structure units.

The document file 50C corresponds to text data describing a protein, a primary structure and a high-order structure of the protein, and the like and text data describing a group and a primary structure of the group, and the like.

The document compression file 51C is a file obtained by encoding the document file 50C in word units.

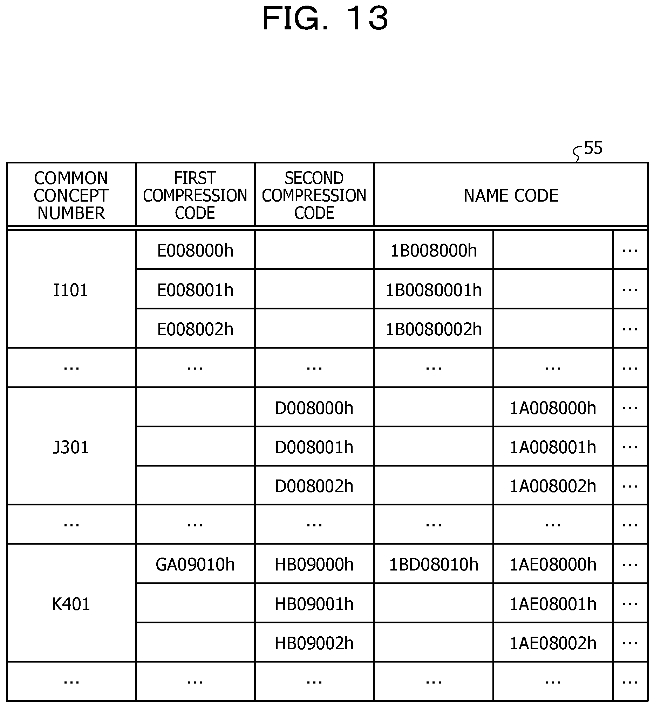

The common concept table 55 is a table that defines the information regarding the genome space S1, the chemical space S2, and the text space S3 to be the common concept. FIG. 13 is a diagram illustrating an example of a data structure of a common concept table. As illustrated in FIG. 13, the common concept table 55 associates a common concept number, a first compression code, a second compression code, and a name code. The first compression code, the second compression code, and the name code associated with the same common concept number are information (compression code) to be classified into the same common concept.

The first compression code is a compression code obtained by compressing information unique to the genome space S1. For example, the first compression code corresponds to a compression code of a protein, a compression code of a primary structure, and a compression code of a high-order structure.

The second compression code is a compression code obtained by compressing information unique to the chemical space S2. For example, the second compression code corresponds to a compression code of a group and a compression code of a group primary structure.

The name code is a compression code obtained by compressing information unique to the text space S3. The information regarding the text space S3 includes a compression code of a name of a protein, a compression code of a name of a primary structure, a compression code of a name of a high-order structure, a compression code of a name of a group, a compression code of a name of a group primary structure, and the like.

In FIG. 13, the first compression codes "E008000h, E008001h, and E00802h" and the name codes "1B008000h, 1B008001h, and 1B008002h" are classified into the same common concept number "I101", and the second compression codes "D008000h, D00801h, and D00802h" and the name codes "1A008000h, 1A008001h, and 1A008002h" are classified into the same common concept number "J301". To the codes classified into the same common concept number, similar vectors are assigned through Poincare Embeddings. For example, compression codes of cimetidine, famotidine, ranitidine, and the like that are antagonists to be bind to histamine H₂ receptors and have similar properties are registered in the common concept table.

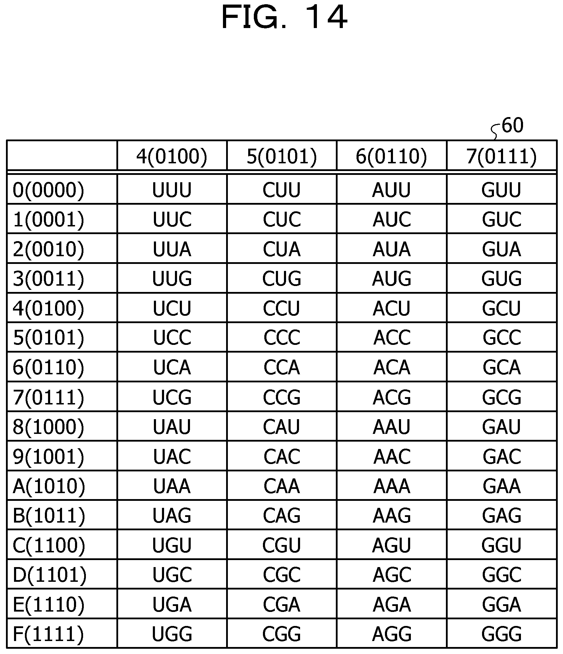

The conversion table 60 is a table that associates a codon and a code of the codon. A group of three base sequences is referred to as a "codon". FIG. 14 is a diagram illustrating an example of a data structure of a conversion table. As illustrated in FIG. 14, each codon and each code are associated. For example, a code of a codon "UUU" is "40h (01000000)". A reference character "h" indicates a hexadecimal.

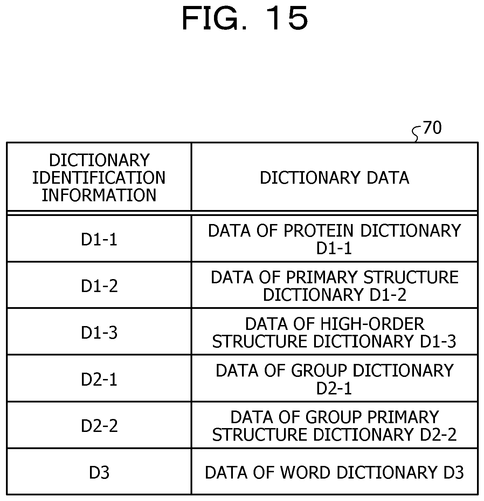

The dictionary table 70 is a table that holds various types of dictionary data described with reference to FIGs. 2 to 4 and the like. FIG. 15 is a diagram illustrating an example of a data structure of a dictionary table. As illustrated in FIG. 15, the dictionary table 70 associates dictionary identification information with the dictionary data. The dictionary identification information is information that uniquely identifies a dictionary. The dictionary data is data of the dictionary. For example, the dictionary data includes data of the protein dictionary D1-1, the primary structure dictionary D1-2, and the high-order structure dictionary D1-3. Furthermore, the dictionary data includes data of the group dictionary D2-1, the group primary structure dictionary D2-2, and the word dictionary D3. In the following, an example of a data structure of each dictionary will be described.

A data structure of the protein dictionary is as illustrated in FIG. 8. As illustrated in FIG. 8, the protein dictionary D1-1 associates a compression code, a name, an amino acid code sequence, and a codon code sequence. The compression code is a compression code assigned to a protein. The name is a name of the protein. The amino acid code sequence is a sequence of amino acid compression codes corresponding to the compression code of the protein. The codon code sequence is a sequence of codon compression codes corresponding to the compression code of the protein.

For example, a compression code "E008000h" is assigned to a protein "type 1 collagen". An amino acid code sequence corresponding to the compression code "E008000h" is "02h46h59h...03h". Furthermore, a codon code sequence corresponding to the compression code "E008000h" is "02h63h78h...03h".

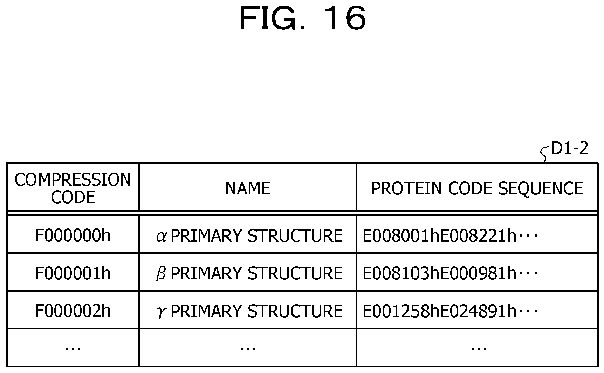

FIG. 16 is a diagram illustrating an example of a data structure of a primary structure dictionary. As illustrated in FIG. 16, the primary structure dictionary D1-2 associates a compression code, a name, and a protein code sequence. The compression code is a compression code assigned to the primary structure of the protein. The name is a name of the primary structure. The protein code sequence is a sequence of protein compression codes corresponding to the primary structure.

For example, a compression code "F000000h" is assigned to a primary structure "α primary structure". A protein code sequence corresponding to the compression code "F000000h" is "E008001hE00822h...".

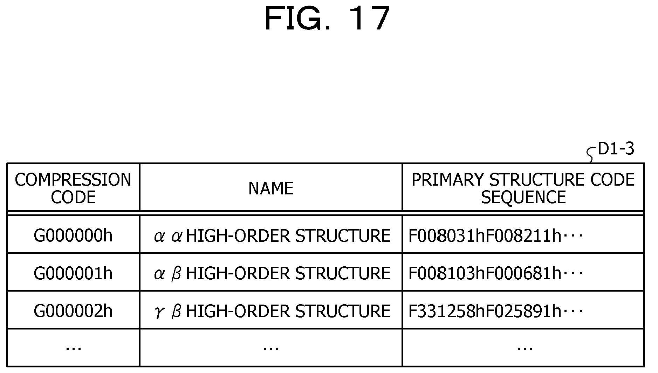

FIG. 17 is a diagram illustrating an example of a data structure of a high-order structure dictionary. As illustrated in FIG. 17, the high-order structure dictionary D1-3 associates a compression code, a name, and a primary structure code sequence. The compression code is a compression code assigned to the high-order structure. The name is a name of the high-order structure. The primary structure code sequence is a sequence of the compression codes of the primary structures corresponding to the high-order structure.

For example, a compression code "G000000h" is assigned to a high-order structure "αα high-order structure". A primary structure code sequence corresponding to the compression code "G000000h" is "F008031hE00821h...".

A data structure of the group dictionary is as illustrated in FIG. 11. As illustrated in FIG. 11, the group dictionary D2-1 associates a compression code, a group, a name, and a rational formula. The compression code is a compression code assigned to the group. The name is an example of a name of the group. The group indicates a group to which the group belongs. The name example is an example of the name of the group. The rational formula indicates a sequence to be a rational formula of the group.

For example, a compression code "D008000h" is assigned to a "methyl group". A rational formula corresponding to the compression code "D008000h" is "CH3".

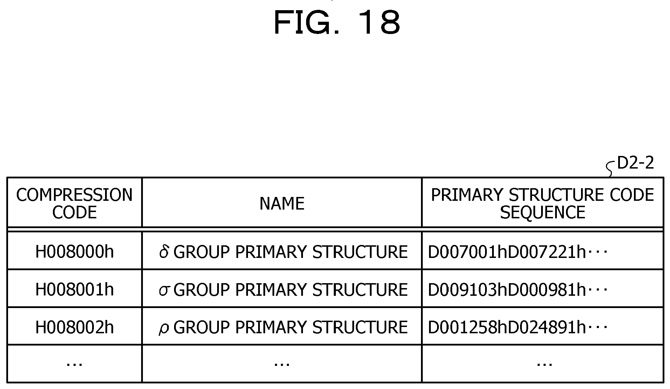

FIG. 18 is a diagram illustrating an example of a data structure of a group primary dictionary. As illustrated in FIG. 18, the group primary structure dictionary D2-2 associates a compression code, a name, and a group code sequence. The compression code is a compression code assigned to the group primary structure. The name is a name of the group primary structure. The group code sequence is a sequence of group compression codes corresponding to the group primary structure.

For example, a compression code "H008000h" is assigned to a "δ group primary structure". A group code sequence corresponding to the compression code "H008000h" is "D007001hD007221h".

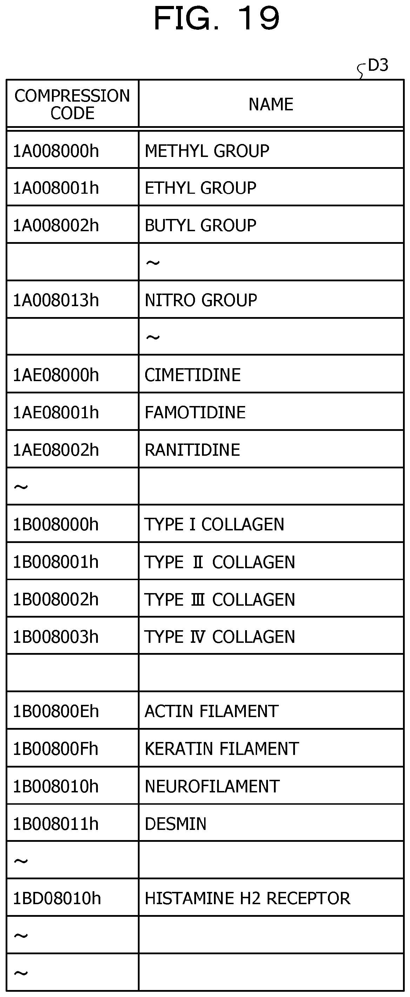

FIG. 19 is a diagram illustrating an example of a data structure of a word dictionary. As illustrated in FIG. 19, the word dictionary D3 associates a compression code with a name. The compression code is a compression code assigned to a word. The name of a name of the word. For example, the word includes the name of the protein, the name of the primary structure, the name of the high-order structure, the name of the functional group, and the name of the group primary structure.

For example, a compression code "1A008000h" is assigned to a "methyl group". The compression code "1B008000h" is assigned to a "type 1 collagen".

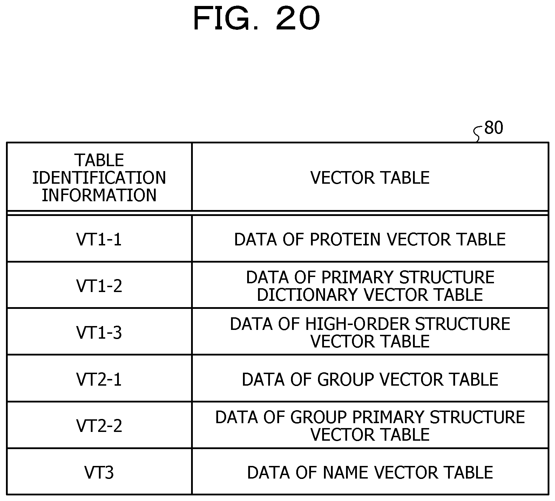

The description returns to FIG. 6. The vector table 80 is a table that holds vectors of the protein, the primary structure, the high-order structure, the group, the group primary structure, and the name. FIG. 20 is a diagram illustrating an example of a data structure of a vector table. As illustrated in FIG. 20, the vector table associates table identification information with the vector table. The table identification information is information that uniquely identifies the vector table.

A vector table of table identification information "VT1-1" is a "protein vector table". In the following description, the vector table of the table identification information "VT1-1" is referred to as a protein vector table VT1-1.

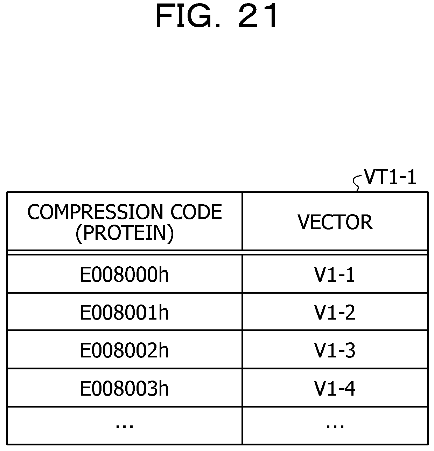

FIG. 21 is a diagram illustrating an example of a data structure of a protein vector table. As illustrated in FIG. 21, the protein vector table VT1-1 associates a compression code of a protein with a vector assigned to the compression code of the protein. The vector of the protein is calculated through Poincare Embeddings.

A vector table of table identification information "VT1-2" is a "primary structure vector table". In the following description, the vector table of the table identification information "VT1-2" is referred to as a primary structure vector table VT1-2.

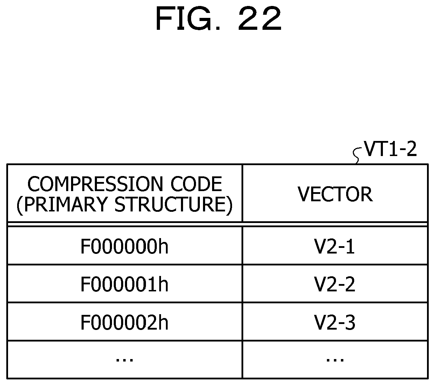

FIG. 22 is a diagram illustrating an example of a data structure of a primary structure vector table. As illustrated in FIG. 22, the primary structure vector table VT1-2 associates a compression code of a primary structure with a vector assigned to the compression code of the primary structure. The vector of the primary structure is calculated by adding the vectors of the proteins included in the primary structure.

A vector table of table identification information "VT1-3" is a "high-order structure vector table". In the following description, the vector table of the table identification information "VT1-3" is referred to as a high-order structure vector table VT1-3.

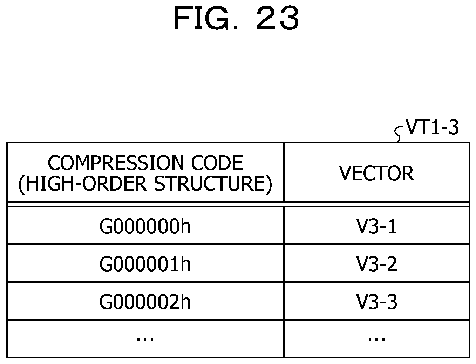

FIG. 23 is a diagram illustrating an example of a data structure of a high-order structure vector table. As illustrated in FIG. 23, the high-order structure vector table VT1-3 associates a compression code of a high-order structure with a vector assigned to the compression code of the high-order structure. The vector of the high-order structure is calculated by adding the vectors of the primary structures included in the high-order structure.

A vector table of table identification information "VT2-1" is a "group vector table". In the following description, the vector table of the table identification information "VT2-1" is referred to as a group vector table VT2-1.

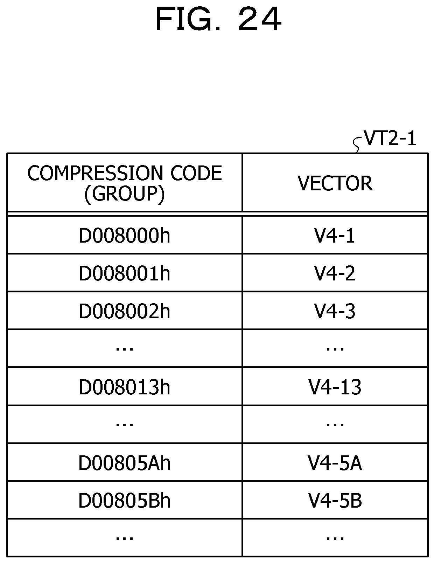

FIG. 24 is a diagram illustrating an example of a data structure of a group vector table. As illustrated in FIG. 24, the group vector table VT2-1 associates a compression code of a group with a vector assigned to the compression code of the group. The vector of the group is calculated through Poincare Embeddings.

A vector table of table identification information "VT2-2" is a "group primary structure vector table". In the following description, the vector table of the table identification information "VT2-2" is referred to as a group primary structure vector table VT2-2.

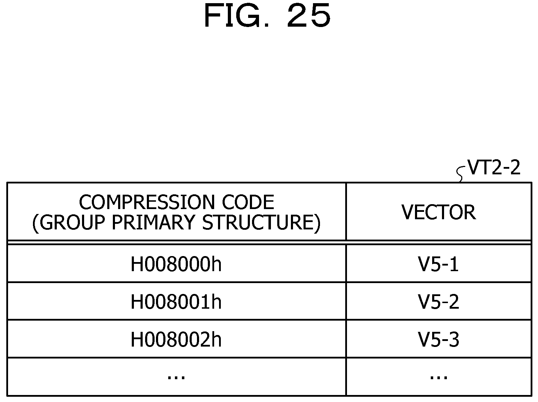

FIG. 25 is a diagram illustrating an example of a data structure of a group primary structure vector table. As illustrated in FIG. 25, the group primary structure vector table VT2-2 associates a compression code of a group primary structure with a vector assigned to the compression code of the group primary structure. The vector of the group primary structure is calculated by adding the vectors of the groups included in the primary structure.

A vector table of table identification information "VT3" is a "name vector table". In the following description, the vector table of the table identification information "VT3" is referred to as a name vector table VT3.

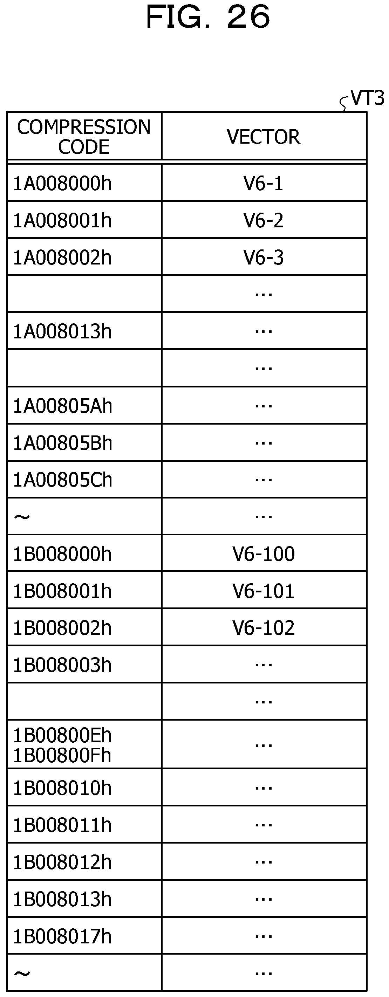

FIG. 26 is a diagram illustrating an example of a data structure of a name vector table. As illustrated in FIG. 26, the name vector table VT3 associates a compression code of a name with a vector assigned to the compression code of the name. The vector of the name is calculated through Poincare Embeddings.

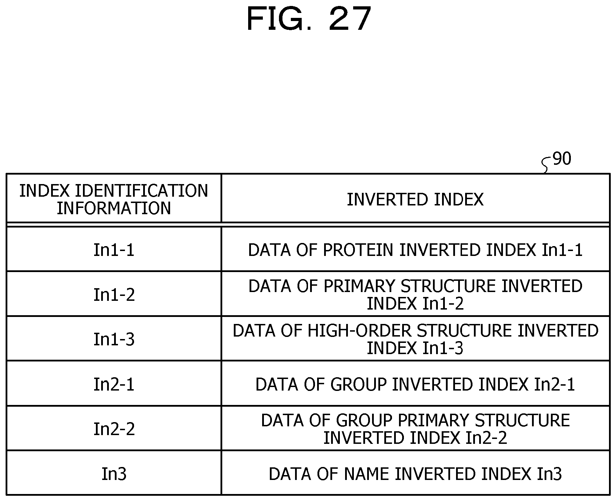

The description returns to FIG. 6. The inverted index table 90 is a table that holds the inverted indexes In1 to In3 described in FIG. 5 and the like. FIG. 27 is a diagram illustrating an example of a data structure of the inverted index table. As illustrated in FIG. 27, the inverted index table 90 associates index identification information with an inverted index. The index identification information is information that uniquely identifies the inverted index. The inverted index is an inverted index that defines a relationship between a vector and a position.

An inverted index corresponding to inverted index identification information "In1-1" corresponds to the protein inverted index In1-1. An inverted index corresponding to index identification information "In 1-2" corresponds to the primary structure inverted index In1-2. An inverted index corresponding to index identification information "In1-3" corresponds to the high-order structure inverted index In1-3.

An inverted index corresponding to index identification information "In2-1" corresponds to the group inverted index In2-1. An inverted index corresponding to index identification information "In2-2" corresponds to the group primary structure inverted index In2-2. An inverted index corresponding to index identification information "In3" corresponds to the name inverted index In3.

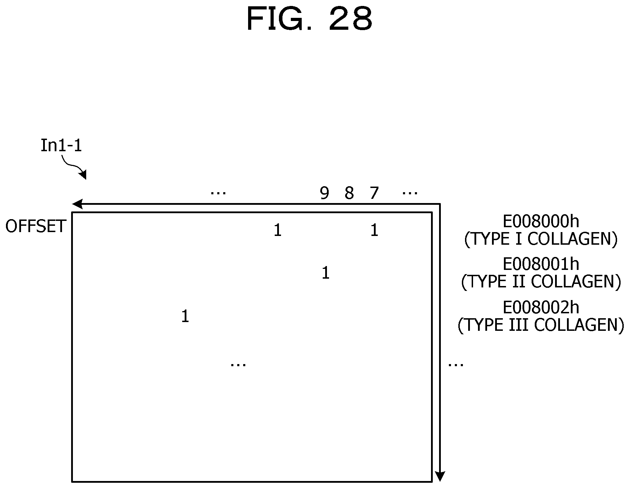

FIG. 28 is a diagram illustrating an example of a data structure of a protein inverted index. A horizontal axis of the protein inverted index In1-1 is an axis corresponding to an offset. A vertical axis of the protein inverted index In1-1 is an axis corresponding to the compression code of the protein. The protein inverted index In1-1 is indicated by a bitmap of "0" or 1", and all bitmaps are set to "0" in an initial state.

For example, it is assumed that an offset of a compression code of a top protein of the protein compressed file 51A be "0". In a case where a code "E008000h (type 1 collagen)" of the protein is included at an eighth position from the beginning of the protein compressed file 51A, a bit at a position where a column of an offset "7" in the protein inverted index In 1-1 intersects with a row of the code "E008000h (type 1 collagen)" of the protein is "1".

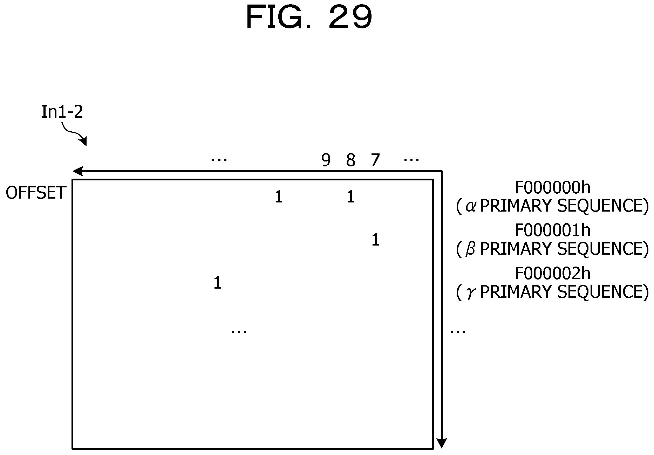

FIG. 29 is a diagram illustrating an example of a data structure of a primary structure inverted index. A horizontal axis of the primary structure inverted index In1-2 is an axis corresponding to an offset. A vertical axis of the primary structure inverted index In1-2 is an axis corresponding to the compression code of the primary structure. The primary structure inverted index In1-2 is indicated by a bitmap of "0" or "1", and all bitmaps are set to "0" in the initial state.

For example, it is assumed that an offset of a compression code of a top primary structure of the primary structure compressed file 52A be "0". In a case where a code "F00000h (a primary structure)" of the primary structure is included at an eighth position from the beginning of the primary structure compressed file 52A, a bit of a position where a column of an offset "7" in the primary structure inverted index In1-2 intersects with a row of the compression code "F000000h (a primary structure)" of the primary structure is "1".

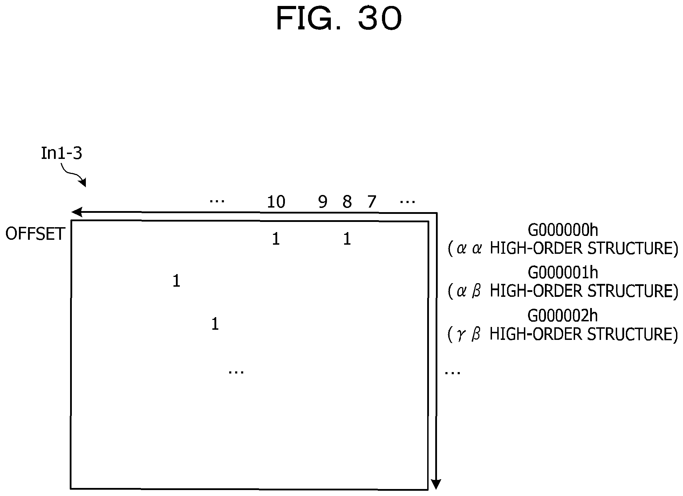

FIG. 30 is a diagram illustrating an example of a data structure of a high-order structure inverted index. A horizontal axis of the high-order structure inverted index In1-3 is an axis corresponding to an offset. A vertical axis of the high-order structure inverted index In1-3 is an axis corresponding to the compression code of the high-order structure. The high-order structure inverted index In1-3 is indicated by a bitmap of "0" or "1", and all bitmaps are set to "0" in the initial state.

For example, it is assumed that an offset of a compression code of a top primary structure of the high-order structure compressed file 53A be "0". In a case where a code "G000000h (αα high-order structure)" of the high-order structure is included at a 11-th position from the beginning of the high-order structure compressed file 53A, a bit at a position where a column of an offset "10" in the high-order structure inverted index In1-3 intersects with a row of the compression code "G000000h (αα high-order structure)" of the high-order structure is "1".

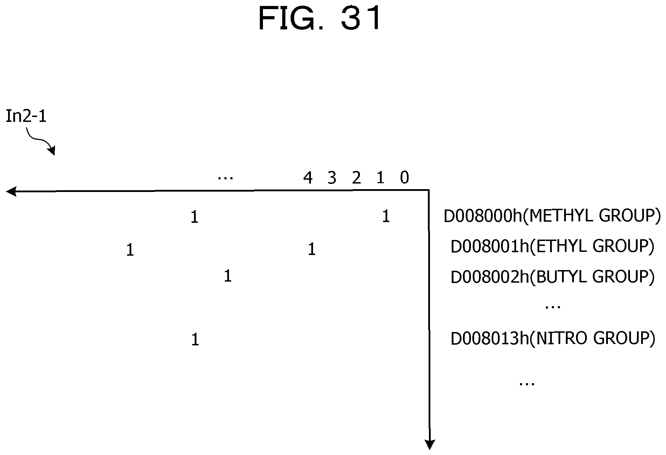

FIG. 31 is a diagram illustrating an example of a data structure of a group inverted index. A horizontal axis of the group inverted index In2-1 is an axis corresponding to an offset. A vertical axis of the group inverted index In2-1 is an axis corresponding to the compression code of the group. The group inverted index In2-1 is indicated by a bitmap of "0" or "1", and all bitmaps are set to "0" in the initial state.

For example, it is assumed that an offset of a compression code of a top group of the group compressed file 51B be "0". In a case where a code "D00800h (methyl group)" of the group is included at a second position from the beginning of the group compressed file 51B, a bit at a position where a column of an offset "1" in the group inverted index In2-1 intersects with a row of the compression code "D008000h (methyl group)" of the group is "1".

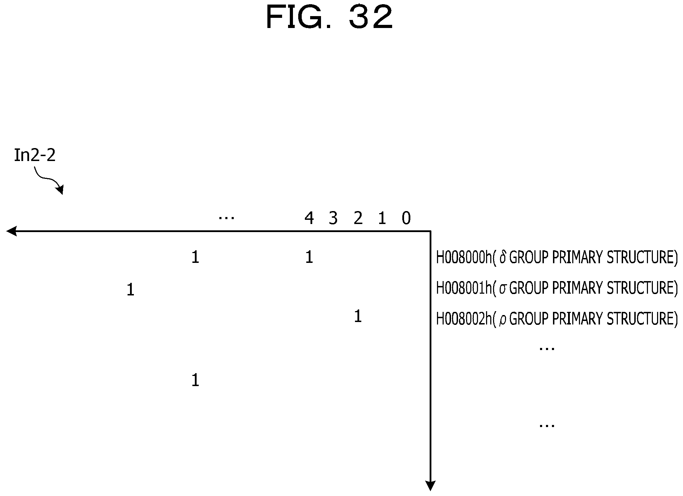

FIG. 32 is a diagram illustrating an example of a data structure of a group primary structure inverted index. A horizontal axis of the group primary structure inverted index In2-2 is an axis corresponding to an offset. A vertical axis of the group primary structure inverted index In2-2 is an axis corresponding to the compression code of the group primary structure. The group primary structure inverted index In2-2 is indicated by a bitmap of "0" or "1", and all bitmaps are set to "0" in the initial state.

For example, it is assumed that an offset of a compression code of a top group primary structure of the group primary structure compressed file 52B be "0". In a case where a code "H00800h (δ group primary structure)" of the group primary structure is included at a fifth position from the beginning of the group primary structure compressed file 52B, a bit at a position where a column of an offset "4" in the group primary structure inverted index In2-2 intersects with a row of the compression code "H008000h (δ group primary structure)" of the group primary structure is "1".

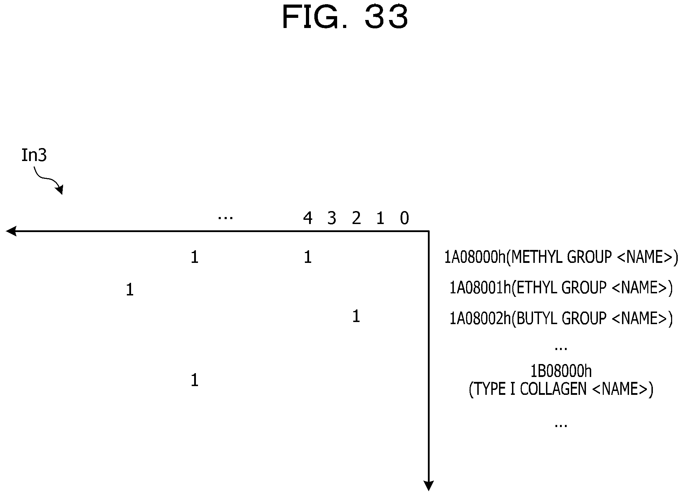

FIG. 33 is a diagram illustrating an example of a data structure of a name inverted index. A horizontal axis of the name inverted index In3 is an axis corresponding to an offset. A vertical axis of the name inverted index In3 is an axis corresponding to the compression code of the name. The name inverted index In3 is indicated by a bitmap of "0" or "1", and all bitmaps are set to "0" in the initial state.

For example, it is assumed that an offset of a compression code of a top name of the document compression file 51C be "0". In a case where a code of a name "1A0800h (methyl group)" is included at a fifth position from the beginning of the document compression file 51C, a bit at a position where a column of an offset "4" in the name inverted index In3 intersects with a row of the compression code "1A08000h (methyl group)" of the name is "1".

Note that, although the position of the compression code is indicated for each compression code in each of the inverted indexes illustrated in FIGs. 28 to 33, indexes of compression codes corresponding to similar vectors may be grouped. For example, in a case where a distance between the vector of the compression code "E008000h" illustrated in FIG. 28 and the vector of the compression code "E008001h" is less than a threshold, a bit string corresponding to the row of the compression code "E008000h" and a bit string corresponding to the row of the compression code "E008001h" may be merged.

The description returns to FIG. 6. The control unit 150 includes an acquisition unit 151, a compression unit 152, a calculation unit 153, a generation unit 154, and a search unit 155. The control unit 150 is implemented by, for example, a central processing unit (CPU) or a micro processing unit (MPU). Furthermore, the control unit 150 may be executed by, for example, an integrated circuit such as an application specific integrated circuit (ASIC) or a field programmable gate array (FPGA).

The acquisition unit 151 is a processing unit that acquires various types of information from an external device via a network. For example, the acquisition unit 151 acquires the base file 50A, the chemical structural formula file 50B, and the document file 50C and registers the acquired base file 50A, chemical structural formula file 50B, and document file 50C in the storage unit 140. The acquisition unit 151 may acquire other information from the external device and register the other information in the storage unit 140.

The compression unit 152 is a processing unit that compresses the base file 50A, the chemical structural formula file 50B, and the document file 50C by assigning compression codes to pieces of information of the base file 50A, the chemical structural formula file 50B, and the document file 50C.

The calculation unit 153 is a processing unit that assigns a vector to the compression code.

The generation unit 154 is a processing unit that generates an inverted index in which a vector assigned to a compression code is associated with a position of the compression code.

The compression unit 152, the calculation unit 153, and the generation unit 154 described above cooperatively operate and generate various types of data as described below.

The compression unit 152 compares the base file 50A with the conversion table 60, assigns a compression code to a base sequence in the base file 50A in codon units, and generates the codon compressed file 50D. The compression unit 152 compares the codon compressed file 50D with the protein dictionary D1-1, assigns a compression code to a sequence of the compression codes of the codons included in the codon compressed file 50D in protein units, and generates the protein compressed file 51A.

The compression unit 152 compares the chemical structural formula file 50B with the group dictionary D2-1, assigns a compression code to a rational formula in the chemical structural formula file 50B in group units, and generates the group compressed file 51B.

The compression unit 152 compares the document file 50C with the word dictionary D3, assigns a compression code to a character string in the document file 50C in word (name) units, and generates the document compression file 51C.

Here, when the protein compressed file 51A, the group compressed file 51B, and the document compression file 51C are generated by the compression unit 152, the calculation unit 153 calculates vectors to be assigned to the compression code of the protein, the compression code of the group, and the compression code of the name.

The calculation unit 153 calculates the vector for each of the compression codes by embedding (performing Poincare Embeddings) the compression code of the protein, the compression code of the group, and the compression code of the name into the same Poincare space P. A vector according to a position of the Poincare space P is assigned to the compression code. In a case of performing Poincare Embeddings, the calculation unit 153 refers to the common concept table 55 and performs adjustment so that compression codes corresponding to the same common concept number are embedded in similar positions in the Poincare space.

The calculation unit 153 outputs a relationship between the compression code and the vector assigned to the compression code to the generation unit 154. The generation unit 154 generates each vector table based on the relationship between the compression code and the vector.

For example, the generation unit 154 generates the protein vector table T1-1 by associating the compression code of the protein with the vector of the compression code. The generation unit 154 registers the protein vector table T1-1 in the vector table 80. The generation unit 154 specifies a relationship between the compression code of the protein and an offset of the compression code in the protein compressed file 51A and generates the protein inverted index In1-1. The generation unit 154 registers the protein inverted index In1-1 in the inverted index table 90.

The generation unit 154 generates the group vector table T2-1 by associating the compression code of the group with the vector of the compression code. The generation unit 154 registers the group vector table T2-1 in the vector table 80. The generation unit 154 specifies a relationship between the compression code of the group and an offset of the compression code in the group compressed file 51B and generates the group inverted index In2-1. The generation unit 154 registers the group inverted index In2-1 in the inverted index table 90.

The generation unit 154 generates the name vector table T3 by associating the compression code of the name (word) with the vector of the compression code. The generation unit 154 generates the name vector table T3 in the vector table 80. The generation unit 154 specifies a relationship between the compression code of the name (word) and an offset of the compression code in the document compression file 51C and generates the name inverted index In3. The generation unit 154 registers the name inverted index In3 in the inverted index table 90.

Subsequently, the compression unit 152 compares the protein compressed file 51A with the primary structure dictionary D1-2, assigns a compression code to a sequence of the protein compression codes included in the protein compressed file 51A in primary structure units, and generates the primary structure compressed file 52A.

The compression unit 152 compares the group compressed file 51B with the group primary structure dictionary D2-2, assigns a compression code to a sequence of the group compression codes included in the group compressed file 51B in group primary structure units, and generates the group primary structure compressed file 52B.

Here, when the primary structure compressed file 52A and the group primary structure compressed file 52B are generated by the compression unit 152, the calculation unit 153 calculates vectors to be assigned to the compression code of the primary structure and the compression code of the group primary structure.

An example of processing for calculating a vector of a compression code of a primary structure by the calculation unit 153 will be described. The calculation unit 153 refers to the primary structure dictionary D1-2 and specifies a protein code sequence (sequence of protein compression codes) corresponding to the compression code of the primary structure. The calculation unit 153 acquires a vector of the specified compression code of each protein from the protein vector table T1-1 and adds the acquired vectors so as to calculate a vector of the compression code of the primary structure.

The calculation unit 153 calculates each vector of the compression code of each primary structure by repeatedly executing the processing described above. Here, in a case where compression code of the primary structure is compared with the common concept table 55 and there are the compression codes of the primary structures belonging to the same common concept number, the calculation unit 153 may correct the compression code.

For example, the calculation unit 153 assumes a vector obtained by averaging the vectors of the compression codes of the primary structures belonging to the same common concept number as a corrected vector. The calculation unit 153 assumes that a compression code "F0000000h" and a compression code "F0000020h" of the primary structure belong to the same common concept number. In this case, the calculation unit 153 assumes a vector obtained by averaging a vector of the compression code "F0000000h" and a vector of the compression code "F0000020h" as a vector of the compression code "F0000000h" and the compression code "F0000020h".

An example of processing for calculating a vector of a compression code of a group primary structure by the calculation unit 153 will be described. The calculation unit 153 refers to the group primary structure dictionary D2-2 and specifies a group code sequence (sequence of group compression codes) corresponding to the compression code of the group primary structure. The calculation unit 153 acquires a vector of the specified compression code of each group from the group vector table T2-1 and adds each of the acquired vectors so as to calculate the vector of the compression code of the group primary structure.

The calculation unit 153 calculates each vector of the compression code of each group primary structure by repeatedly executing the processing described above.

The calculation unit 153 outputs a relationship between the compression code of the primary structure and the vector assigned to the compression code to the generation unit 154. Furthermore, a relationship between the compression code of the group primary structure and the vector assigned to the compression code is output to the generation unit 154. The generation unit 154 generates each vector table based on the relationship between the compression code and the vector.

For example, the generation unit 154 generates the primary structure vector table T1-2 by associating the compression code of the primary structure with the vector of the compression code. The generation unit 154 registers the primary structure vector table T1-2 in the vector table 80. The generation unit 154 specifies a relationship between the compression code of the primary structure and an offset of the compression code in the primary structure compressed file 52A and generates the primary structure inverted index In1-2. The generation unit 154 generates the primary structure inverted index In1-2 in the inverted index table 90.

The generation unit 154 generates the group primary structure vector table T2-2 by associating the compression code of the group primary structure with the vector of the compression code. The generation unit 154 registers the group primary structure vector table T2-2 in the vector table 80. The generation unit 154 specifies a relationship between the compression code of the group primary structure with an offset of the compression code in the group primary structure compressed file 52B and generates the group primary structure inverted index In2-2. The generation unit 154 registers the group primary structure inverted index In2-2 in the inverted index table 90.

Subsequently, the compression unit 152 compares the primary structure compressed file 52A with the high-order structure dictionary D1-3, assigns a compression code to a sequence of the compression codes of the primary structures included in the primary structure compressed file 52A in high-order structure units, and generates the high-order structure compressed file 53A.

Here, when the high-order structure compressed file 53A is generated by the compression unit 152, the calculation unit 153 calculates a vector to be assigned to the compression code of the high-order structure.

An example of processing for calculating a vector of a compression code of a high-order structure by the calculation unit 153 will be described. The calculation unit 153 refers to the high-order structure dictionary D1-3 and specifies a primary structure code sequence (sequence of primary structure compression codes) corresponding to the compression code of the high-order structure. The calculation unit 153 acquires a vector of the specified compression code of each primary structure from the primary structure vector table T1-2 and adds each of the acquired vectors so as to calculate the vector of the compression code of the high-order structure.

The calculation unit 153 calculates each vector of the compression code of each high-order structure by repeatedly executing the processing described above. The calculation unit 153 outputs a relationship between the compression code of the high-order structure and the vector assigned to the compression code to the generation unit 154.

The generation unit 154 generates the high-order structure vector table T1-3 by associating the compression code of the high-order structure with the vector of the compression code. The generation unit 154 registers the high-order structure vector table T1-3 in the vector table 80. The generation unit 154 specifies a relationship between the compression code of the high-order structure and an offset of the compression code in the high-order structure compressed file 53A and generates the high-order structure inverted index In1-3. The generation unit 154 registers the high-order structure inverted index In1-3 in the inverted index table 90.

By executing the processing described above, the compression unit 152 generates the codon compressed file 50D, the protein compressed file 51A, the primary structure compressed file 52A, and the high-order structure compressed file 53A from the base file 50A. The compression unit 152 generates the group compressed file 51B and the group primary structure compressed file 52B from the chemical structural formula file 50B. The compression unit 152 generates the document compression file 51C from the document file 50C.

The calculation unit 153 calculates the vector of the compression code of the protein, the vector of the compression code of the primary structure, and the vector of the compression code of the high-order structure. The calculation unit 153 calculates the vector of the compression code of the group and the vector of the compression code of the group primary structure. The calculation unit 153 calculates the vector of the compression code of the name.

The generation unit 154 generates the vector table 80 and the inverted index table 90.

The description returns to FIG. 6. The search unit 155 is a processing unit that searches the storage unit 140 for information corresponding to a search query in a case of receiving the search query and outputs a search result. The search unit 155 evaluates a similarity between a vector of the search query and the vectors of the inverted indexes In1 to In3. For example, the search unit 155 specifies a vector of which a distance from the vector of the search query is less than a threshold as the similar vector.

The search unit 155 acquires information regarding a protein, a primary structure, or a high-order structure corresponding to the similar vector based on the similar vector and the inverted index In1, from the protein compressed file 51A, the primary structure compressed file 52A, and the high-order structure compressed file 53A. The information processing device acquires information regarding a group or a primary structure corresponding to the similar vector, based on the similar vector and the inverted index In2, from the group compressed file 51B and the group primary structure compressed file 52B. The information processing device acquires a name (or document data related to name) corresponding to the similar vector, based on the similar vector and the inverted index In3, from the document compression file 51C.

The search unit 155 outputs the acquired information as the search result for the search query. In a case of receiving the search query from the input unit 120, the search unit 155 outputs the search result to the display unit 130 and displays the search result on the display unit 130. Since the information acquired by the search unit 155 is encoded, the information may be decoded using the dictionary table 70, and the decoded search result may be output.

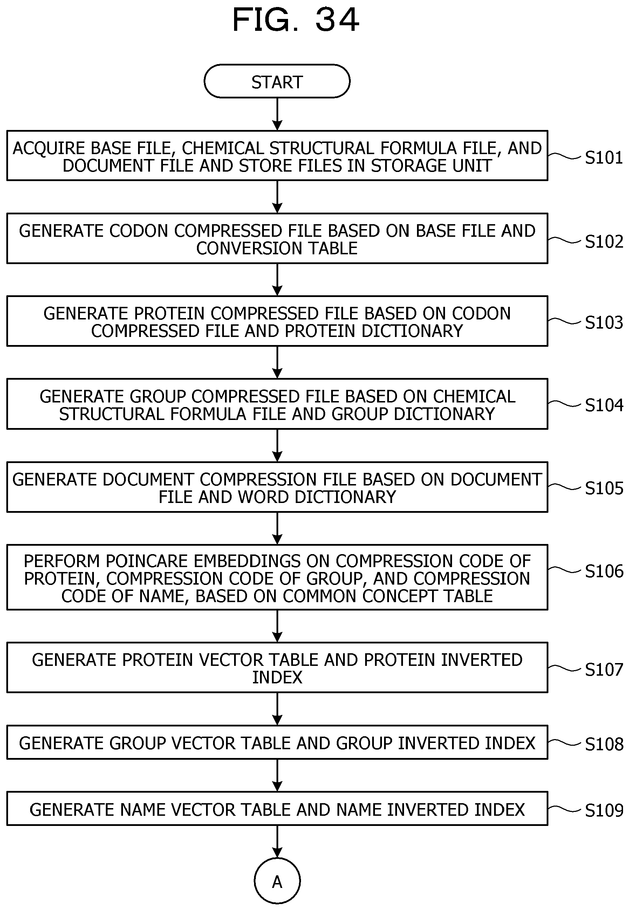

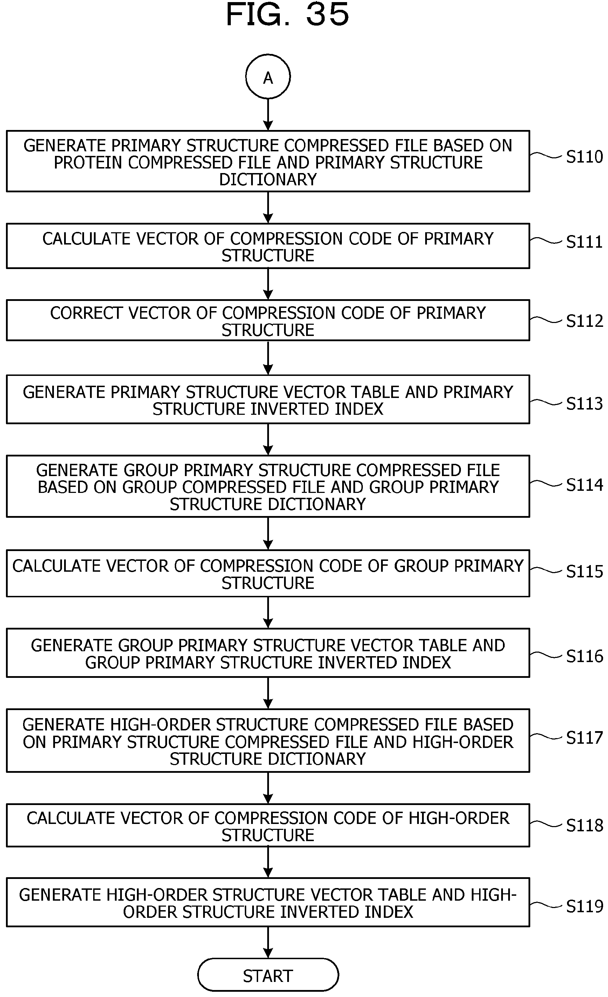

Next, an example of a processing procedure of the information processing device 100 according to the first embodiment will be described. FIGs. 34 and 35 are flowcharts illustrating a processing procedure of the information processing device according to the first embodiment. FIG. 34 will be described. The acquisition unit 151 of the information processing device 100 acquires the base file 50A, the chemical structural formula file 50B, and the document file 50C and registers the acquired files in the storage unit 140 (step S101).

The compression unit 152 of the information processing device 100 generates the codon compressed file 50D based on the base file 50A and the conversion table 60 (step S102). The compression unit 152 generates the group compressed file 51B based on the chemical structural formula file 50B and the group dictionary D2-1 (step S103). The compression unit 152 generates the document compression file 51C based on the document file 50C and the word dictionary D3 (step S104).

The compression unit 152 generates the document compression file 51C based on the document file 50C and the word dictionary D3 (step S105). The calculation unit 153 of the information processing device 100 performs Poincare Embeddings on the compression code of the protein, the compression code of the group, and the compression code of the name, based on the common concept table 55 (step S106).

The generation unit 154 of the information processing device 100 generates the protein vector table T1-1 and the protein inverted index In1-1 (step S107). The generation unit 154 generates the group vector table T2-1 and the group inverted index In2-1 (step S108).

The generation unit 154 generates the name vector table T3 and the name inverted index In3 (step S109) and proceeds to step S110 in FIG. 35.

The description proceeds to FIG. 35. The compression unit 152 generates the primary structure compressed file 52A based on the protein compressed file 51A and the primary structure dictionary D1-2 (step S110). The calculation unit 153 calculates a vector of a compression code of a primary structure (step S111). The calculation unit 153 corrects the vector of the compression code of the primary structure (step S112).

The generation unit 154 generates the primary structure vector table T1-2 and the primary structure inverted index In1-2 (step S113). The compression unit 152 generates the group primary structure compressed file 52B based on the group compressed file 51B and the group primary structure dictionary D2-2 (step S114).

The calculation unit 153 calculates a vector of a compression code of a group primary structure (step S115). The generation unit 154 generates the primary structure vector table T2-2 and the group primary structure inverted index In2-2 (step S116).

The compression unit 152 generates the high-order structure compressed file 53A based on the primary structure compressed file 52A and the high-order structure dictionary D1-3 (step S117). The calculation unit 153 calculates a vector of a compression code of a high-order structure (step S118). The generation unit 154 generates the high-order structure vector table T1-3 and the high-order structure inverted index In1-3 (step S119).

Next, effects of the information processing device 100 according to the first embodiment will be described. The information processing device 100 performs Poincare Embeddings on information regarding a plurality of spaces based on the common concept table 55 and assigns a vector. The information processing device 100 generates the inverted indexes In1 to In3 in which the vector assigned to the information is associated with a position of the information. In a case of receiving a search query, the information processing device 100 acquires information similar to the search query from the plurality of spaces based on the vector of the search query and the inverted indexes In1 to In3 as a search result. As a result, similarity search can be accurately performed with various granularities such as high-order structures of compounds, primary structures, or proteins.

The information processing device 100 calculates a vector of structural information with a larger granularity than the basic information based on the vector of the basic information on which Poincare Embeddings has been performed. As a result, the vector of the structural information with a larger granularity can be accurately calculated.

For example, the information processing device 100 specifies a protein included in a primary structure based on the primary structure dictionary D1-2 and adds vectors of the proteins included in the primary structure so as to calculate a vector of the primary structure. The information processing device 100 specifies the primary structure included in a high-order structure and adds vectors of the primary structures included in the high-order structure so as to calculate a vector of the high-order structure. As a result, the vectors of the primary structure and the high-order structure can be accuracy calculated.

The information processing device 100 specifies a group included in a group primary structure based on the group primary structure dictionary D2-2 and adds vectors of the groups included in the group primary structure so as to calculate a vector of the group primary structure. As a result, the vector of the group primary structure can be accurately calculated.

The information processing device 100 generates the protein inverted index In1-1 in which the vector of the protein and the position of the protein in the protein compressed file 51A are associated. The information processing device 100 generates the primary structure inverted index In1-2 in which the vector of the primary structure and the position of the primary structure in the primary structure compressed file 52A are associated. The information processing device 100 generates the high-order structure inverted index In1-3 in which the vector of the high-order structure and the position of the high-order structure in the high-order structure compressed file 53A are associated. As a result, search can be performed across with granularities of the protein, the primary structure, and the high-order structure.

The information processing device 100 generates the group inverted index In2-1 in which the vector of the group and the position of the group in the group compressed file 51B are associated. The information processing device 100 generates the group primary structure inverted index In2-2 in which the vector of the group primary structure and the position of the group primary structure in the group primary structure compressed file 52B are associated. As a result, search can be performed across with granularities of the group and the group primary structure.

By the way, as described above, the calculation unit 153 of the information processing device 100 according to the first embodiment calculates the vector by performing Poincare Embeddings on the compression code of the protein, the compression code of the group, and the compression code of the name, based on the common concept table 55. Then, the calculation unit 153 calculates the vector of the primary structure by adding the compression codes of the plurality of proteins, and calculates the vector of the compression code of the high-order structure by adding the vectors of the compression codes of the plurality of primary structures. However, the present invention is not limited to this.

The calculation unit 153 of the information processing device 100 may directly calculate the vector of the compression code of the high-order structure by performing Poincare Embeddings on the compression code of the high-order structure. In a case of performing Poincare Embeddings on the compression code of the high-order structure, the calculation unit 153 specifies the compression codes of the high-order structures belonging to the same common concept number based on the common concept table 55 and adjusts an embedding position so as to embed the specified compression codes of the high-order structures at positions in the Poincare space P close to each other. As a result, the similar vectors can be calculated for the compression codes of the high-order structures belonging to the same common concept number.

Furthermore, after calculating the vector corresponding to the compression code of the high-order structure through Poincare Embeddings, the calculation unit 153 may specify the compression codes of the plurality of primary structures included in the high-order structure based on the high-order structure dictionary D1-3 and assign vectors to the specified compression codes of the respective primary structures.

After assigning the vector to the compression code of each primary structure, the calculation unit 153 may correct the vector of the compression code of the same primary structure based on the vector assigned to the compression code of the same primary structure.

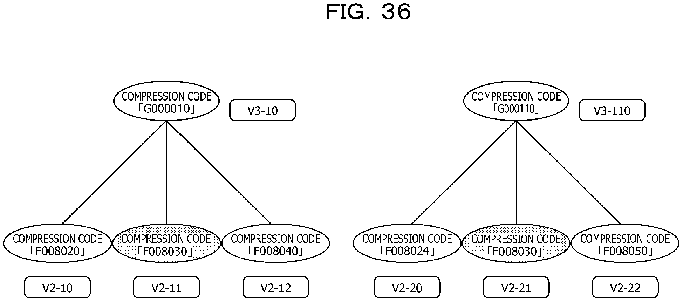

FIG. 36 is a diagram for explaining another processing of the calculation unit. In FIG. 36, the calculation unit 153 calculates a vector by performing Poincare Embeddings on compression codes "G000010" and "G000110" of a high-order structure. For example, it is assumed that a vector of the compression code "G000010" be "V3-10" and a vector of the compression code "G000110" be "V3-11".

It is assumed that compression codes of primary structures corresponding to the compression code "G000010" of the high-order structure be "F008020", "F008030", and "F008040" and vectors of the compression codes of the respective primary structures be "V2-10", "V2-11", and "V2-12". For example, the vector of the compression code of each primary structure is calculated from the vector assigned to the compression code of the high-order structure.

It is assumed that compression codes of primary structures corresponding to the compression code "G000110" of the high-order structure be "F008024", "F008030", and "F008050" and vectors of the compression codes of the respective primary structures be "V2-20", "V2-21", and "V2-22". For example, the vector of the compression code of each primary structure is calculated from the vector assigned to the compression code of the high-order structure.

Here, the calculation unit 153 determines that the compression code (1) "F008030" included in the compression code "G000010" and the compression code (2) "F008030" included in the compression code "G000110" are the same compression code. In this case, the calculation unit 153 averages vectors of respective dimensions including the vector "V2-11" of the compression code (1) "F008030" and the vector "V2-21" of the compression code (2) "F008030" and calculates each optimized vector value as the vector of the compression code "F008030".

By executing the processing described above by the calculation unit 153, it is possible to improve accuracy of the vector of the primary structure.

Furthermore, the information processing device 100 described above may perform Poincare Embeddings using the common concept table 55 that focuses on ligands (agonists) and antagonists that have similar functions with respect to a receptor, regarding the granularity of the high-order structure. In other words, the same common concept number is assigned to a compression code to be assigned to the ligand and a compression code of the antagonist that have similar functions, among the compression codes of the high-order structures.

### [Second Embodiment]

Next, processing of an information processing device according to a second embodiment will be described. The information processing device according to the second embodiment performs machine learning using teacher data indicating a relationship between a receptor and a ligand or a relationship between a receptor and an antagonist and learns a learning model. In a case where the learning model is learned, the receptor, the ligand, and the antagonist included in the teacher data are contained into vectors, and learning is performed. The receptor, the ligand, and the antagonist are compounds with a high-order structure, and the information processing device specifies a vector using the high-order structure vector table generated in the first embodiment.

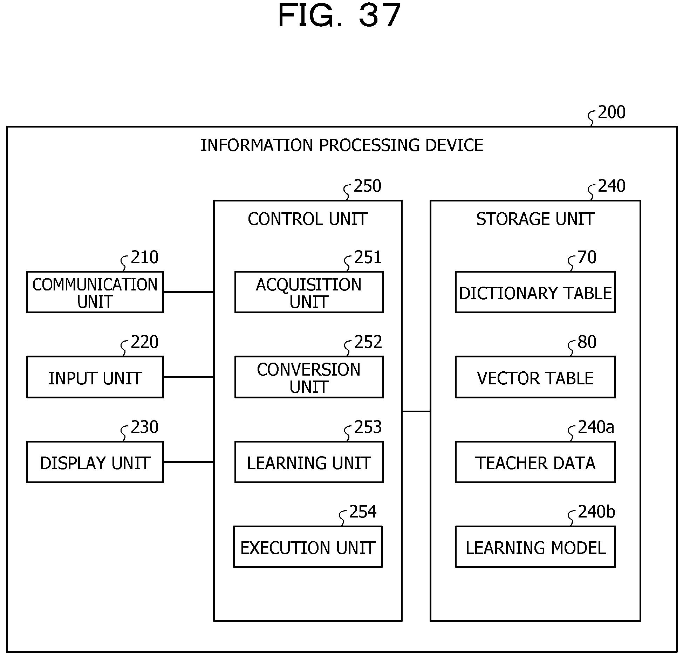

FIG. 37 is a functional block diagram illustrating a configuration of the information processing device according to the second embodiment. As illustrated in FIG. 37, this information processing device 200 includes a communication unit 210, an input unit 220, a display unit 230, a storage unit 240, and a control unit 250.

Descriptions of the communication unit 210, the input unit 220, and the display unit 230 are similar to the descriptions of the communication unit 210, the input unit 220, and the display unit 230 in the first embodiment.

The storage unit 240 includes a dictionary table 70, a vector table 80, teacher data 240a, and a learning model 240b. The storage unit 240 is implemented by, for example, a semiconductor memory element such as a RAM or a flash memory, or a storage device such as a hard disk or an optical disk.

The dictionary table 70 corresponds to the dictionary table 70 described with reference to FIG. 15. The dictionary table 70 includes data of a protein dictionary D1-1, a primary structure dictionary D1-2, and a high-order structure dictionary D1-3. Furthermore, the dictionary table 70 includes data of a group dictionary D2-1, a group primary structure dictionary D2-2, and a word dictionary D3.

The vector table 80 corresponds to the vector table 80 described with reference to FIG. 20. The vector table 80 includes a protein vector table VT1-1, a primary structure dictionary vector table VT1-2, and a high-order structure vector table VT1-3. Furthermore, the vector table 80 includes a group vector table VT2-1, a group primary structure vector table VT2-2, and a name vector table VT3.

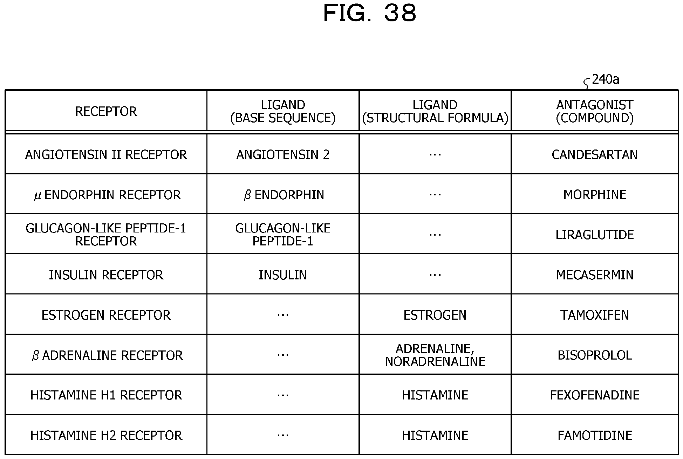

The teacher data 240a is data that defines relationships between a receptor and a ligand and a receptor and an antagonist. FIG. 38 is a diagram illustrating an example of a data structure of teacher data. As illustrated in FIG. 38, the teacher data 240a associates a receptor, a ligand (base sequence), a ligand (structural formula), and an antagonist (compound).

For example, the receptor serves as input data, and the ligand (base sequence), the ligand (chemical structural formula), and the antagonist (compound) serve as correct answer labels. The receptor and the antagonist are high-order structures. A primary structure and a high-order structure of the ligand (base sequence) are specified by a base sequence. A group primary structure of the ligand (chemical structural formula) is specified by a rational formula. Note that the receptor, the ligand, and the antagonist may be specified by names.

The learning model 240b is a machine learning model that is learned by a learning unit 253 to be described later. The learning model 240b is a machine learning model such as a neural network (NN).

The control unit 250 includes an acquisition unit 251, a conversion unit 252, the learning unit 253, and an execution unit 254. The control unit 250 is, for example, implemented by a CPU or an MPU. Furthermore, the control unit 250 may be implemented by, for example, an integrated circuit such as an ASIC or an FPGA.

The acquisition unit 151 is a processing unit that acquires various types of information from an external device via a network. For example, the acquisition unit 151 acquires the teacher data 240a and registers the acquired teacher data 240a in the storage unit 240.

The conversion unit 252 assigns vectors to the input data and the correct answer label included in the teacher data 240a and outputs the vectors assigned to the input data and the correct answer label to the learning unit 253.

For example, in a case where the input data and the correct answer label are specified by the compression code, the conversion unit 252 compares the compression code with the vector table 80 and specifies a vector corresponding to the compression code.

In a case where the input data and the correct answer label are specified by the name, the conversion unit 252 compares the word dictionary D3 with the name and specifies a compression code corresponding to the name, and then, specifies a vector corresponding to the compression code using the vector table 80.

In a case where the input data and the correct answer label are specified by the base sequence, the conversion unit 252 specifies a compression code of the high-order structure using the dictionary table 70, and then, specifies a vector corresponding to the compression code using the vector table 80. Processing for converting the base sequence into the compression code of the high-order structure is similar to the processing executed by the compression unit 152 in the first embodiment.

In a case where the input data and the correct answer label are specified by the chemical structural formula, the conversion unit 252 specifies a compression code of the group primary structure using the dictionary table 70, and then, specifies a vector corresponding to the compression code using the vector table 80. Processing for converting the chemical structural formula (rational formula) into the compression code of the group primary structure is similar to the processing executed by the compression unit 152 in the first embodiment.

The learning unit 253 is a processing unit that machine learns the learning model 240b using a relationship between a vector of the input data of the teacher data 240a and a vector of the correct answer label. For example, the learning unit 253 acquires output data in a case where the vector of the input data is input to the NN (learning model 240b). The learning unit 253 sets a difference between the output data and the correct answer label (vector) as a loss function, back-propagates an error of the loss function, and learns a parameter of the learning model 240b so that a value of the loss function is minimized. The learning unit 253 stores the learning model 240b to be a learning result in the storage unit 240.

The execution unit 254 is a processing unit that, in a case of receiving a search query corresponding to the receptor, searches for a ligand or an antagonist corresponding to the search query by inputting the search query into the learning model 240b. For example, the execution unit 254 acquires the output data (vector) by inputting the search query (vector) into the learning model 240b.

The execution unit 254 compares a vector of the output data with the vector of the name vector table VT3, specifies a compression code of a name corresponding to the vector of the most similar name, and specifies a name of the ligand or the antagonist corresponding to the output data based on the specified compression code and the dictionary table 70.

Although description is omitted in FIG. 37, the storage unit 240 of the information processing device 200 may further store information stored in the storage unit 140 in FIG. 6. The execution unit 254 may detect a base sequence and a rational formula corresponding to the ligand or the antagonist corresponding to the vector of the output data by executing the processing similar to the search unit 155 and output the base sequence and the rational formula as detection results.

Furthermore, although the description is omitted in FIG. 37, the information processing device 200 may include functional units (compression unit 152, calculation unit 153, and generation unit 154) as in the information processing device 100. The compression unit 152, the calculation unit 153, and the generation unit 154 included in the information processing device 200 may generate the vector table 80 and the inverted index table 90.

Next, effects of the information processing device 200 according to the second embodiment will be described. The information processing device 200 generates the learning model 240b based on the teacher data 240a that defines the relationship between the receptor and the ligand or between the receptor and the antagonist. As a result, in a case where the search query is given, it is possible to specify the ligand or the antagonist corresponding to the search query (receptor) by inputting the search query into the learning model 240b.

Here, since the receptor associated in the teacher data 240a is the primary structure and the antagonist is the group, granularities are different. However, the vectors can be assigned as described above, and the relationship between the receptor and the antagonist can be learned. Furthermore, vectors of similar primary structures may be grouped. In this way, by grouping the vectors of the similar primary structures, the number of combinations of the teacher data can be reduced. Furthermore, as a result, a computational explosion of a Softmax function can be prevented, and it is possible to perform AI estimation with a graphics processing unit (GPU) with a low performance.

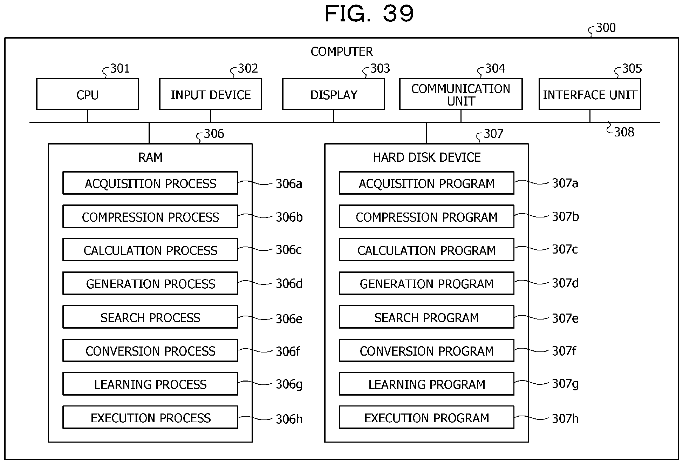

Next, an example of a hardware configuration of a computer that implements functions similar to those of the information processing device 100 (200) described in the embodiments described above will be described. FIG. 39 is a diagram illustrating an example of a hardware configuration of a computer that implements functions similar to those of the information processing device according to the embodiments.

As illustrated in FIG. 39, a computer 300 includes a CPU 301 that executes various types of arithmetic processing, an input device 302 that receives an input of data from a user, and a display 303. Furthermore, the computer 300 includes a communication device 304 that exchanges data with an external device and the like via a wired or wireless network, and an interface device 305. Furthermore, the computer 300 includes a RAM 306 that temporarily stores various types of information, and a hard disk device 307. Then, each of the devices 301 to 307 is connected to a bus 308.

The hard disk device 307 includes an acquisition program 307a, a compression program 307b, a calculation program 307c, a generation program 307d, a search program 307e, a conversion program 307f, a learning program 307g, and an execution program 307h. Furthermore, the CPU 301 reads each of the programs 307a to 307h and develops the programs to the RAM 306.

The acquisition program 307a functions as an acquisition process 306a. The compression program 307b functions as a compression process 306b. The calculation program 307c functions as a calculation process 306c. The generation program 307d functions as a generation process 306d. The search program 307e functions as a search process 306e. The conversion program 307f functions as a conversion process 306f. The learning program 307g functions as a learning process 306g. The execution program 307h functions as an execution process 306h.

Processing of the acquisition process 306a corresponds to the processing of the acquisition units 151 and 251. Processing of the compression process 306b corresponds to the processing of the compression unit 152. Processing of the calculation process 306c corresponds to the processing of the calculation unit 153. Processing of the generation process 306d corresponds to the processing of the generation unit 154. Processing of the search process 306e corresponds to the processing of the search unit 155. Processing of the conversion process 306f corresponds to the processing of the conversion unit 252. Processing of the learning process 306g corresponds to the processing of the learning unit 253. Processing of the execution process 306h corresponds to the processing of the execution unit 254.

Note that each of the programs 307a to 307h does not necessarily have to be stored in the hard disk device 307 from the beginning. For example, each of the programs is stored in a "portable physical medium" to be inserted in the computer 300, such as a flexible disk (FD), a compact disc read only memory (CD-ROM), a digital versatile disc (DVD), a magneto-optical disk, or an IC card. Then, the computer 300 may read the respective programs 307a to 307h to execute them.

### CITATION LIST

### PATENT DOCUMENT

Patent Document 1: International Publication Pamphlet No. WO 2007/139037

### NON-PATENT DOCUMENT

Non-Patent Document 1: Anna Klimovskaia et al. "Poincaré maps for analyzing complex hierarchies in single-cell data" nature communications

### REFERENCE SIGNS LIST

50Abase file
50Bchemical structural formula file
50Cdocument file
50Dcodon compressed file
51Aprotein compressed file
51Bgroup compressed file
51Cdocument compression file
52Aprimary structure compressed file
52Bgroup primary structure compressed file
53Ahigh-order structure compressed file
55common concept table
60conversion table
70dictionary table
80vector table
90inverted index table
100, 200information processing device
110, 210communication unit
120, 220input unit
130, 230display unit
140, 240storage unit
150, 250control unit
151, 251acquisition unit
152compression unit
153calculation unit
154generation unit
155search unit
252conversion unit
253learning unit
254execution unit

## Claims

1. An information processing program comprising instructions which, when executed by a computer, cause the computer to perform processing comprising:
calculating vectors of a plurality of pieces of space-specific basic information defined in a plurality of spaces, the plurality of spaces comprising a genome space, a chemical space and a text space, by performing Poincare Embeddings on the plurality of pieces of basic information, based on a common concept table that classifies the plurality of pieces of basic information with a common concept and calculate a vector of structural information with a granularity larger than the basic information by adding vectors of the plurality of pieces of basic information on which the Poincare Embeddings have been performed and of which pieces of basic information are included in the structural information;
generating an inverted index that defines a relationship between a position of the basic information in a file that corresponds to the same space and the vector of the basic information and a relationship between a position of the structural information in the file and the vector of the structural information; and
in response to receiving a search query, evaluating a similarity between a vector of the search query and vectors of the inverted indexes, searching for a similar vector, the similar vector being a vector similar to the vector of the search query from the vectors of the inverted indexes, acquiring information regarding pieces of basic information corresponding to the similar vector, and outputting the acquired information as a search result for the search query.

2. The information processing program according to claim **1,** wherein the calculating of the vectors includes calculating a vector of each of first basic information specific for a first space defined in the first space, second basic information specific for a second space defined in the second space, and third basic information defined in a third space by performing Poincare Embeddings on the first basic information, the second basic information, and the third basic information, based on a common concept table that classifies the first basic information, the second basic information, and the third basic information with a common concept.

3. The information processing program according to claim **2,** wherein the generating of the inverted index includes calculating a vector of first structural information with a granularity larger than the first basic information in the first space, based on the vectors of a plurality of pieces of the first basic information and generates a first inverted index in which a position of the first basic information in a file of the first space, a position of the vector of the first structural information, and the vector are associated.

4. The information processing program according to claim **3,** wherein the generating of the inverted index includes correcting the vectors of a plurality of pieces of similar first structural information, based on the vectors of the plurality of pieces of similar first structural information.

5. The information processing program according to claim **2,** wherein the first space is a genome space that uses a protein as the first basic information, the second space is a chemical space that uses a functional group as the second basic information, and the third space is a text space that uses a name of the protein or the functional group as the third basic information.

6. The information processing program according to claim **3,** the processing further comprising:
calculating a vector of a receptor, a vector of a ligand, and a vector of an antagonist that belong to the first structural information, regarding teacher data that uses the receptor as input data and one of the ligand or the antagonist as a correct answer label and generate a learning model, based on the vector of the receptor, the vector of the ligand, and the vector of the antagonist, by setting a difference between output data and the correct answer label as a loss function, back-propagating an error of the loss function, and learning a parameter of the learning model so that a value of the loss function is minimized;
in response to receiving a search query, inputting the vector of the search query corresponding to a receptor into the learning model to acquire a vector of output data, detecting a base sequence and a rational formula corresponding to a ligand or an antagonist corresponding to the vector of the output data, and outputting the base sequence and the rational formula as detection results.

7. An information processing method implemented by a computer, the information processing method comprising:
calculating vectors of a plurality of pieces of space-specific basic information defined in a plurality of spaces, the plurality of spaces comprising a genome space, a chemical space and a text space, by performing Poincare Embeddings on the plurality of pieces of basic information, based on a common concept table that classifies the plurality of pieces of basic information with a common concept and calculate a vector of structural information with a granularity larger than the basic information by adding vectors of the plurality of pieces of basic information on which the Poincare Embeddings have been performed and of which pieces of basic information are included in the structural information;
generating an inverted index that defines a relationship between a position of the basic information in a file that corresponds to the same space and the vector of the basic information and a relationship between a position of the structural information in the file and the vector of the structural information; and
in response to receiving a search query, evaluating a similarity between a vector of the search query and vectors of the inverted indexes, searching for a similar vector, the similar vector being a vector similar to the vector of the search query from the vectors of the inverted indexes, acquiring information regarding pieces of basic information corresponding to the similar vector, and outputting the acquired information as a search result for the search query.

8. The information processing method according to claim 7, wherein the calculating of the vectors includes calculating a vector of each of first basic information specific for a first space defined in the first space, second basic information specific for a second space defined in the second space, and third basic information defined in a third space by performing Poincaré Embeddings on the first basic information, the second basic information, and the third basic information, based on a common concept table that classifies the first basic information, the second basic information, and the third basic information with a common concept.

9. The information processing method according to claim 8, wherein the generating of the inverted index includes calculating a vector of first structural information with a granularity larger than the first basic information in the first space, based on the vectors of a plurality of pieces of the first basic information and generates a first inverted index in which a position of the first basic information in a file of the first space, a position of the vector of the first structural information, and the vector are associated.

10. The information processing method according to claim 9, wherein the generating of the inverted index includes correcting the vectors of a plurality of pieces of similar first structural information, based on the vectors of the plurality of pieces of similar first structural information.

11. The information processing method according to claim 8, wherein the first space is a genome space that uses a protein as the first basic information, the second space is a chemical space that uses a functional group as the second basic information, and the third space is a text space that uses a name of the protein or the functional group as the third basic information.

12. The information processing method according to claim 9, the processing further comprising:
calculating a vector of a receptor, a vector of a ligand, and a vector of an antagonist that belong to the first structural information, regarding teacher data that uses the receptor as input data and one of the ligand or the antagonist as a correct answer label and generate a learning model, based on the vector of the receptor, the vector of the ligand, and the vector of the antagonist, by setting a difference between output data and the correct answer label as a loss function, back-propagating an error of the loss function, and learning a parameter of the learning model so that a value of the loss function is minimized;
in response to receiving a search query, inputting the vector of the search query corresponding to a receptor into the learning model to acquire a vector of output data, detecting a base sequence and a rational formula corresponding to a ligand or an antagonist corresponding to the vector of the output data, and outputting the base sequence and the rational formula as detection results.

13. An information processing device (100, 200) comprising:
a calculation unit (153) configured to calculate vectors of a plurality of pieces of space-specific basic information defined in a plurality of spaces, the plurality of spaces comprising a genome space, a chemical space and a text space, by performing Poincaré Embeddings on the plurality of pieces of basic information, based on a common concept table that classifies the plurality of pieces of basic information with a common concept and calculate a vector of structural information with a granularity larger than the basic information by adding vectors of the plurality of pieces of basic information on which the Poincaré Embeddings have been performed and of which pieces of basic information are included in the structural information;
a generation unit (154) configured to generate an inverted index that defines a relationship between a position of the basic information in a file that corresponds to the same space and the vector of the basic information and a relationship between a position of the structural information in the file and the vector of the structural information; and
a search unit (155) configured to, in response to receiving a search query, evaluate a similarity between a vector of the search query and vectors of the inverted indexes, search for a similar vector, the similar vector being a vector similar to the vector of the search query from the vectors of the inverted indexes, acquire information regarding pieces of basic information corresponding to the similar vector, and output the acquired information as a search result for the search query.

14. The information processing device (100, 200) according to claim 13, wherein the calculation unit (153) calculates a vector of each of first basic information specific for a first space defined in the first space, second basic information specific for a second space defined in the second space, and third basic information defined in a third space by performing Poincaré Embeddings on the first basic information, the second basic information, and the third basic information, based on a common concept table that classifies the first basic information, the second basic information, and the third basic information with a common concept.

15. The information processing device (100, 200) according to claim 14, wherein the generation unit (154) calculates a vector of first structural information with a granularity larger than the first basic information in the first space, based on the vectors of a plurality of pieces of the first basic information and generates a first inverted index in which a position of the first basic information in a file of the first space, a position of the vector of the first structural information, and the vector are associated.

16. The information processing device (100, 200) according to claim 15, wherein the generation unit (154) corrects the vectors of a plurality of pieces of similar first structural information, based on the vectors of the plurality of pieces of similar first structural information.

17. The information processing device (100, 200) according to claim 14, wherein the first space is a genome space that uses a protein as the first basic information, the second space is a chemical space that uses a functional group as the second basic information, and the third space is a text space that uses a name of the protein or the functional group as the third basic information.

18. The information processing device (100, 200) according to claim 15, further comprising:
a learning unit (253) configured to calculate a vector of a receptor, a vector of a ligand, and a vector of an antagonist that belong to the first structural information, regarding teacher data that uses the receptor as input data and one of the ligand or the antagonist as a correct answer label and generate a learning model, based on the vector of the receptor, the vector of the ligand, and the vector of the antagonist, by setting a difference between output data and the correct answer label as a loss function, back-propagating an error of the loss function, and learning a parameter of the learning model so that a value of the loss function is minimized;
an execution unit (254) configured to, in response to receiving a search query, input the vector of the search query corresponding to a receptor into the learning model to acquire a vector of output data, detect a base sequence and a rational formula corresponding to a ligand or an antagonist corresponding to the vector of the output data, and output the base sequence and the rational formula as detection results.

## Patentansprüche

1. Informationsverarbeitungsprogramm, umfassend Anweisungen, die, wenn sie von einem Computer ausgeführt werden, bewirken, dass der Computer Verarbeitung durchführt, die Folgendes umfasst:
Berechnen von Vektoren einer Vielzahl von Teilen raumspezifischer Grundinformationen, die in einer Vielzahl von Räumen definiert sind, wobei die Vielzahl von Räumen einen Genomraum, einen chemischen Raum und einen Textraum umfasst, durch Durchführen von Poincaré-Einbettungen an der Vielzahl von Teilen von Grundinformationen basierend auf einer gemeinsamen Konzepttabelle, die die Vielzahl von Teilen von Grundinformationen mit einem gemeinsamen Konzept klassifiziert und einen Vektor von Strukturinformationen mit einer Granularität, die größer ist als die Grundinformationen, berechnet durch Hinzufügen von Vektoren der Vielzahl von Teilen von Grundinformationen an denen die Poincaré-Einbettungen durchgeführt wurden und von denen Teile von Grundinformationen in den Strukturinformationen enthalten sind;
Erzeugen eines invertierten Index, der eine Beziehung zwischen einer Position der Grundinformationen in einer Datei definiert, die dem gleichen Raum und dem Vektor der Grundinformationen und einer Beziehung zwischen einer Position der Strukturinformationen in der Datei und dem Vektor der Strukturinformationen entspricht; und
als Reaktion auf Empfangen einer Suchanfrage Beurteilen einer Ähnlichkeit zwischen einem Vektor der Suchanfrage und Vektoren der invertierten Indizes, Suchen nach einem ähnlichen Vektor, wobei der ähnliche Vektor ein Vektor ist, der dem Vektor der Suchanfrage unter den Vektoren der invertierten Indizes ähnlich ist, Erfassen von Informationen in Bezug auf Teile von Grundinformationen, die dem ähnlichen Vektor entsprechen, und Ausgeben der erfassten Informationen als ein Suchergebnis für die Suchanfrage.

2. Informationsverarbeitungsprogramm nach Anspruch 1, wobei das Berechnen der Vektoren Berechnen eines Vektors von jeder von ersten Grundinformationen einschließt, die spezifisch für einen ersten Raum sind, die im ersten Raum definiert sind, von zweiten Grundinformationen, die spezifisch für einen zweiten Raum sind, die im zweiten Raum definiert sind, und von dritten Grundinformationen, die in einem dritten Raum definiert sind, durch Durchführen von Poincaré-Einbettungen an den ersten Grundinformationen, den zweiten Grundinformationen und den dritten Grundinformationen basierend auf einer gemeinsamen Konzepttabelle, die die ersten Grundinformationen, die zweiten Grundinformationen und die dritten Grundinformationen mit einem gemeinsamen Konzept klassifiziert.

3. Informationsverarbeitungsprogramm nach Anspruch 2, wobei das Erzeugen des invertierten Index Berechnen eines Vektors von ersten Strukturinformationen mit einer Granularität, die größer ist als die ersten Grundinformationen in dem ersten Raum, einschließt, basierend auf den Vektoren einer Vielzahl von Teilen der ersten Grundinformationen, und einen ersten invertierten Index erzeugt, in dem eine Position der ersten Grundinformationen in einer Datei des ersten Raums, eine Position des Vektors der ersten Strukturinformationen und der Vektor zugeordnet sind.

4. Informationsverarbeitungsprogramm nach Anspruch 3, wobei das Erzeugen des invertierten Index Korrigieren der Vektoren einer Vielzahl von Teilen von ähnlichen ersten Strukturinformationen basierend auf den Vektoren der Vielzahl von Teilen von ähnlichen ersten Strukturinformationen einschließt.

5. Informationsverarbeitungsprogramm nach Anspruch 2, wobei der erste Raum ein Genomraum ist, der ein Protein als die ersten Grundinformationen verendet, der zweite Raum ein chemischer Raum ist, der eine funktionelle Gruppe als die zweiten Grundinformationen verwendet, und der dritte Raum ein Textraum ist, der einen Namen des Proteins oder der funktionellen Gruppe als die dritten Grundinformationen verwendet.

6. Informationsverarbeitungsprogramm nach Anspruch 3, wobei die Verarbeitung weiter Folgendes umfasst:
Berechnen eines Vektors eines Rezeptors, eines Vektors eines Liganden und eines Vektors eines Antagonisten, die zu den ersten Strukturinformationen gehören, in Bezug auf Lehrerdaten, die den Rezeptor als Eingabedaten und einen des Liganden oder des Antagonisten als ein korrektes Antwortetikett verwenden, und Erzeugen eines Lernmodells basierend auf dem Vektor des Rezeptors, dem Vektor des Liganden und dem Vektor des Antagonisten durch Setzen einer Differenz zwischen Ausgabedaten und dem korrekten Antwortetikett als eine Verlustfunktion, Backpropagation eines Fehlers der Verlustfunktion und Lernen eines Parameters des Lernmodells so, dass ein Wert der Verlustfunktion minimiert wird;
als Reaktion auf Empfangen einer Suchanfrage, Eingeben des Vektors der Suchanfrage, der einem Rezeptor entspricht, in das Lernmodell, um einen Vektor von Ausgabedaten zu erfassen, eine Basissequenz und eine rationale Formel zu erkennen, die einem Liganden oder einem Antagonisten entspricht, die dem Vektor der Ausgabedaten entspricht, und Ausgeben der Basissequenz und der rationalen Formel als Erkennungsergebnis.

7. Informationsverarbeitungsverfahren, das von einem Computer implementiert wird, wobei das Informationsverarbeitungsverfahren Folgendes umfasst:
Berechnen von Vektoren einer Vielzahl von Teilen raumspezifischer Grundinformationen, die in einer Vielzahl von Räumen definiert sind, wobei die Vielzahl von Räumen einen Genomraum, einen chemischen Raum und einen Textraum umfasst, durch Durchführen von Poincaré-Einbettungen an der Vielzahl von Teilen von Grundinformationen basierend auf einer gemeinsamen Konzepttabelle, die die Vielzahl von Teilen von Grundinformationen mit einem gemeinsamen Konzept klassifiziert und einen Vektor von Strukturinformationen mit einer Granularität, die größer ist als die Grundinformationen, berechnet durch Hinzufügen von Vektoren der Vielzahl von Teilen von Grundinformationen an denen die Poincaré-Einbettungen durchgeführt wurden und von denen Teile von Grundinformationen in den Strukturinformationen enthalten sind;
Erzeugen eines invertierten Index, der eine Beziehung zwischen einer Position der Grundinformationen in einer Datei definiert, die dem gleichen Raum und dem Vektor der Grundinformationen und einer Beziehung zwischen einer Position der Strukturinformationen in der Datei und dem Vektor der Strukturinformationen entspricht; und
als Reaktion auf Empfangen einer Suchanfrage, Beurteilen einer Ähnlichkeit zwischen einem Vektor der Suchanfrage und Vektoren der invertierten Indizes, Suchen nach einem ähnlichen Vektor, wobei der ähnliche Vektor ein Vektor ist, der dem Vektor der Suchanfrage unter den Vektoren der invertierten Indizes ähnlich ist, Erfassen von Informationen in Bezug auf Teile von Grundinformationen, die dem ähnlichen Vektor entsprechen, und Ausgeben der erfassten Informationen als ein Suchergebnis für die Suchanfrage.

8. Informationsverarbeitungsverfahren nach Anspruch 7, wobei das Berechnen der Vektoren Berechnen eines Vektors von jeder von ersten Grundinformationen einschließt, die spezifisch für einen ersten Raum sind, die im ersten Raum definiert sind, von zweiten Grundinformationen, die spezifisch für einen zweiten Raum sind, die im zweiten Raum definiert sind, und von dritten Grundinformationen, die einem dritten Raum definiert sind, durch Durchführen von Poincaré-Einbettungen an den ersten Grundinformationen, den zweiten Grundinformationen und den dritten Grundinformationen basierend auf einer gemeinsamen Konzepttabelle, die die ersten Grundinformationen, die zweiten Grundinformationen und die dritten Grundinformationen mit einem gemeinsamen Konzept klassifiziert.

9. Informationsverarbeitungsverfahren nach Anspruch 8, wobei das Erzeugen des invertierten Index Berechnen eines Vektors von ersten Strukturinformationen mit einer Granularität einschließt, die größer ist als die ersten Grundinformationen im ersten Raum basierend auf den Vektoren einer Vielzahl von Teilen der ersten Grundinformationen und einen ersten invertierten Index erzeugt, in dem eine Position der ersten Grundinformationen in einer Datei des ersten Raums, eine Position des Vektors der ersten Strukturinformationen und der Vektor zugeordnet sind.

10. Informationsverarbeitungsverfahren nach Anspruch 9, wobei das Erzeugen des invertierten Index Korrigieren der Vektoren einer Vielzahl von Teilen von ähnlichen ersten Strukturinformationen basierend auf den Vektoren der Vielzahl von Teilen von ähnlichen ersten Strukturinformationen einschließt.

11. Informationsverarbeitungsverfahren nach Anspruch 8, wobei der erste Raum ein Genomraum ist, der ein Protein als die ersten Grundinformationen verwendet, der zweite Raum ein chemischer Raum ist, der eine funktionelle Gruppe als die zweiten Grundinformationen verwendet, und der dritte Raum ein Textraum ist, der einen Namen des Proteins oder der funktionellen Gruppe als die dritten Grundinformationen verwendet.

12. Informationsverarbeitungsverfahren nach Anspruch 9, wobei die Verarbeitung weiter Folgendes umfasst:
Berechnen eines Vektors eines Rezeptors, eines Vektors eines Liganden und einen Vektor eines Antagonisten, die zu den ersten Strukturinformationen gehören, in Bezug auf Lehrerdaten, die den Rezeptor als Eingabedaten und einen des Liganden oder des Antagonisten als ein korrektes Antwortetikett verwenden, und Erzeugen eines Lernmodells basierend auf dem Vektor des Rezeptors, dem Vektor des Liganden und dem Vektor des Antagonisten durch Setzen einer Differenz zwischen Ausgabedaten und dem korrekten Antwortetikett als eine Verlustfunktion, Backpropagation eines Fehlers der Verlustfunktion und Lernen eines Parameters des Lernmodells so, dass ein Wert der Verlustfunktion minimiert wird;
als Reaktion auf Empfangen einer Suchanfrage, Eingeben des Vektors der Suchanfrage, der einem Rezeptor entspricht, in das Lernmodell, um einen Vektor von Ausgabedaten zu erfassen, eine Basissequenz und eine rationale Formel zu erkennen, die einem Liganden oder einem Antagonisten entspricht, die dem Vektor der Ausgabedaten entspricht, und Ausgeben der Basissequenz und der rationalen Formel als Erkennungsergebnis.

13. Informationsverarbeitungsvorrichtung (100, 200), umfassend:
eine Berechnungseinheit (153), die so konfiguriert ist, dass sie Vektoren einer Vielzahl von Teilen raumspezifischer Grundinformationen berechnet, die in einer Vielzahl von Räumen definiert sind, wobei die Vielzahl von Räumen einen Genomraum, einen chemischen Raum und einen Textraum umfasst, durch Durchführen von Poincaré-Einbettungen an der Vielzahl von Teilen von Grundinformationen, basierend auf einer gemeinsamen Konzepttabelle, die die Vielzahl von Teilen von Grundinformationen mit einem gemeinsamen Konzept klassifiziert und einen Vektor von Strukturinformationen mit einer Granularität, die größer ist als die Grundinformationen, berechnet durch Hinzufügen von Vektoren der Vielzahl von Teilen von Grundinformationen an denen die Poincaré-Einbettungen durchgeführt wurden und von denen Teile von Grundinformationen in den Strukturinformationen enthalten sind;
eine Erzeugungseinheit (154), die so konfiguriert ist, dass sie einen invertierten Index erzeugt, der eine Beziehung zwischen einer Position der Grundinformationen in einer Datei, die dem gleichen Raum und dem Vektor der Grundinformationen entspricht, und eine Beziehung zwischen einer Position der Strukturinformationen in der Datei und den Vektor der Strukturinformationen definiert; und
eine Steuereinheit (155), die so konfiguriert ist, dass sie als Reaktion auf Empfangen einer Suchanfrage eine Ähnlichkeit zwischen einem Vektor der Suchanfrage und Vektoren der invertierten Indizes beurteilt und nach einem ähnlichen Vektor sucht, wobei der ähnliche Vektor ein Vektor ist, der dem Vektor der Suchanfrage unter den Vektoren der invertierten Indizes ähnlich ist, Informationen in Bezug auf Teile von Grundinformationen erfasst, die dem ähnlichen Vektor entsprechen, und die erfassten Informationen als ein Suchergebnis für die Suchanfrage ausgibt.

14. Informationsverarbeitungsvorrichtung (100, 200) nach Anspruch 13, wobei die Berechnungseinheit (153) einen Vektor von jeder der ersten Grundinformationen, die spezifisch für einen ersten Raum sind, die im ersten Raum definiert sind, von zweiten Grundinformationen, die spezifisch für einen zweiten Raum sind, die im zweiten Raum definiert sind, und von dritten Grundinformationen, die einem dritten Raum definiert sind, berechnet, durch Durchführen von Poincaré-Einbettungen an den ersten Grundinformationen, den zweiten Grundinformationen und den dritten Grundinformationen basierend auf einer gemeinsamen Konzepttabelle, die die ersten Grundinformationen, die zweiten Grundinformationen und die dritten Grundinformationen mit einem gemeinsamen Konzept klassifiziert.

15. Informationsverarbeitungsvorrichtung (100, 200) nach Anspruch 14, wobei die Erzeugungseinheit (154) einen Vektor von ersten Strukturinformationen mit einer Granularität, die größer ist als die ersten Grundinformationen in dem ersten Raum, basierend auf den Vektoren einer Vielzahl von Teilen der ersten Grundinformationen berechnet und einen ersten invertierten Index erzeugt, in dem eine Position der ersten Grundinformationen in einer Datei des ersten Raums, eine Position des Vektors der ersten Strukturinformationen und der Vektor zugeordnet sind.

16. Informationsverarbeitungsvorrichtung (100, 200) nach Anspruch 15, wobei die Erzeugungseinheit (154) die Vektoren einer Vielzahl von Teilen von ähnlichen ersten Strukturinformationen basierend auf den Vektoren der Vielzahl von Teilen von ähnlichen ersten Strukturinformationen korrigiert.

17. Informationsverarbeitungsvorrichtung (100, 200) nach Anspruch 14, wobei der erste Raum ein Genomraum ist, der ein Protein als die ersten Grundinformationen verwendet, der zweite Raum ein chemischer Raum ist, der eine funktionelle Gruppe als die zweiten Grundinformationen verwendet, und der dritte Raum ein Textraum ist, der einen Namen des Proteins oder der funktionellen Gruppe als die dritten Grundinformationen verwendet.

18. Informationsverarbeitungsvorrichtung (100, 200) nach Anspruch 15, weiter umfassend:
eine Lerneinheit (253), die so konfiguriert ist, dass sie einen Vektor eines Rezeptors, einen Vektor eines Liganden und einen Vektor eines Antagonisten berechnet, die zu den ersten Strukturinformationen gehören, in Bezug auf Lehrerdaten, die den Rezeptor als Eingabedaten und einen des Liganden oder des Antagonisten als ein korrektes Antwortetikett verwenden, und basierend auf dem Vektor des Rezeptors, dem Vektor des Liganden und dem Vektor des Antagonisten ein Lernmodell erzeugt, durch Setzen einer Differenz zwischen Ausgabedaten und dem korrekten Antwortetikett als eine Verlustfunktion, Backpropagation eines Fehlers der Verlustfunktion und Lernen eines Parameters des Lernmodells so, dass ein Wert der Verlustfunktion minimiert wird;
eine Ausführungseinheit (254), die so konfiguriert ist, dass sie als Reaktion auf Empfangen einer Suchanfrage den Vektor der Suchanfrage, der einem Rezeptor entspricht, in das Lernmodell eingibt, um einen Vektor von Ausgabedaten zu erfassen, eine Basissequenz und eine rationale Formel zu erkennen, die einem Liganden oder einem Antagonisten entspricht, die dem Vektor der Ausgabedaten entspricht, und die Basissequenz und die rationale Formel als Erkennungsergebnis ausgibt.

## Revendications

1. Programme de traitement d'informations comprenant des instructions qui, lorsqu'elles sont mises en œuvre par un ordinateur, amènent l'ordinateur à mettre en œuvre un traitement comprenant :
le calcul de vecteurs d'une pluralité d'éléments d'informations de base spécifiques à un espace définies dans une pluralité d'espaces, la pluralité d'espaces comprenant un espace génomique, un espace chimique et un espace textuel,
en mettant en œuvre un plongement de Poincaré sur la pluralité d'éléments d'informations de base, sur la base d'un tableau de concept commun qui classe la pluralité d'éléments d'informations de base
avec un concept commun et calcule un vecteur d'informations structurelles avec une granularité plus élevée que les informations de base en ajoutant des vecteurs de la pluralité d'éléments d'informations de base sur lesquelles le plongement de Poincaré a été mis en œuvre et dont les éléments d'informations de base sont inclus dans les informations structurelles ;
la génération d'un indice inversé qui définit une relation entre une position des informations de base dans un fichier qui correspond au même espace et le vecteur des informations de base et une relation entre une position des informations structurelles dans le fichier et le vecteur des informations structurelles ; et
en réponse à la réception d'une demande de recherche, l'évaluation d'une similarité entre un vecteur de la demande de recherche et des vecteurs des indices inversés, la recherche d'un vecteur similaire, le vecteur similaire étant un vecteur similaire au vecteur de la demande de recherche parmi les vecteurs des indices inversés, l'acquisition d'informations concernant des éléments d'informations de base correspondant au vecteur similaire et la délivrance en sortie des informations acquises en tant que résultat de recherche de la demande de recherche.

2. Programme de traitement d'informations selon la revendication 1, dans lequel le calcul des vecteurs inclut le calcul d'un vecteur de chacune de premières informations de base spécifiques pour un premier espace définies dans le premier espace, de deuxièmes informations de base spécifiques pour un deuxième espace définies dans le deuxième espace, et de troisièmes informations de base définies dans un troisième espace en mettant en œuvre un plongement de Poincaré sur les premières informations de base, les deuxièmes informations de base et les troisièmes informations de base, sur la base d'un tableau de concept commun qui classe les premières informations de base, les deuxièmes informations de base et les troisièmes informations de base avec un concept commun.

3. Programme de traitement d'informations selon la revendication 2, dans lequel la génération de l'indice inversé inclut le calcul d'un vecteur de premières informations structurelles avec une granularité plus élevée que les premières informations de base dans le premier espace, sur la base des vecteurs d'une pluralité d'éléments des premières informations de base et génère un premier indice inversé dans lequel sont associés une position des premières informations de base dans un fichier du premier espace, une position du vecteur des premières informations structurelles et le vecteur.

4. Programme de traitement d'informations selon la revendication 3, dans lequel la génération de l'indice inversé inclut la correction des vecteurs d'une pluralité d'éléments de premières informations structurelles similaires, sur la base des vecteurs de la pluralité d'éléments de premières informations structurelles similaires.

5. Programme de traitement d'informations selon la revendication 2, dans lequel le premier espace est un espace génomique qui utilise une protéine comme premières informations de base, le deuxième espace est un espace chimique qui utilise un groupe fonctionnel comme deuxièmes informations de base et le troisième espace est un espace textuel qui utilise un nom de la protéine ou le groupe fonctionnel comme troisièmes informations de base.

6. Programme de traitement d'informations selon la revendication 3, le traitement comprenant en outre :
le calcul d'un vecteur d'un récepteur, d'un vecteur d'un ligand et d'un vecteur d'un antagoniste qui appartient aux premières informations structurelles, concernant des données d'enseignement qui utilisent le récepteur comme données d'entrée et l'un parmi le ligand ou l'antagoniste comme étiquette de réponse correcte et la génération d'un modèle d'apprentissage, sur la base du vecteur du récepteur, du vecteur du ligand et du vecteur de l'antagoniste, en réglant une différence entre les données de sortie et l'étiquette de réponse correcte comme fonction de perte, en rétro-propageant une erreur de la fonction de perte et en apprenant un paramètre du modèle d'apprentissage de telle sorte qu'une valeur de la fonction de perte soit minimisée ;
en réponse à la réception d'une demande de recherche, l'entrée du vecteur de la demande de recherche correspondant à un récepteur dans le modèle d'apprentissage pour acquérir un vecteur de données de sortie, la détection d'une séquence de base et d'une formule rationnelle correspondant à un ligand ou à un antagoniste correspondant au vecteur des données de sortie, et la délivrance en sortie de la séquence de base et de la formule rationnelle comme résultats de détection.

7. Procédé de traitement d'informations mis en œuvre par ordinateur, le procédé de traitement d'informations comprenant :
le calcul de vecteurs d'une pluralité d'éléments d'informations de base spécifiques à un espace définies dans une pluralité d'espaces, la pluralité d'espaces comprenant un espace génomique, un espace chimique et un espace textuel, en mettant en œuvre un plongement de Poincaré sur la pluralité d'éléments d'informations de base, sur la base d'un tableau de concept commun qui classe la pluralité d'éléments d'informations de base avec un concept commun et calcule un vecteur d'informations structurelles avec une granularité plus élevée que les informations de base en ajoutant des vecteurs de la pluralité d'éléments d'informations de base sur lesquelles le plongement de Poincaré a été mis en œuvre et dont les éléments d'informations de base sont inclus dans les informations structurelles ;
la génération d'un indice inversé qui définit une relation entre une position des informations de base dans un fichier qui correspond au même espace et le vecteur des informations de base et une relation entre une position des informations structurelles dans le fichier et le vecteur des informations structurelles ; et
en réponse à la réception d'une demande de recherche, l'évaluation d'une similarité entre un vecteur de la demande de recherche et des vecteurs des indices inversés, la recherche d'un vecteur similaire, le vecteur similaire étant un vecteur similaire au vecteur de la demande de recherche parmi les vecteurs des indices inversés, l'acquisition d'informations concernant des éléments d'informations de base correspondant au vecteur similaire et la délivrance en sortie des informations acquises en tant que résultat de recherche de la demande de recherche.

8. Procédé de traitement d'informations selon la revendication 7, dans lequel le calcul des vecteurs inclut le calcul d'un vecteur de chacune de premières informations de base spécifiques pour un premier espace définies dans le premier espace, de deuxièmes informations de base spécifiques pour un deuxième espace définies dans le deuxième espace et de troisièmes informations de base définies dans un troisième espace en mettant en œuvre un plongement de Poincaré sur les premières informations de base, les deuxièmes informations de base et les troisièmes informations de base, sur la base d'un tableau de concept commun qui classe les premières informations de base, les deuxièmes informations de base et les troisièmes informations de base avec un concept commun.

9. Procédé de traitement d'informations selon la revendication 8, dans lequel la génération de l'indice inversé inclut le calcul d'un vecteur de premières informations structurelles avec une granularité plus élevée que les premières informations de base dans le premier espace, sur la base des vecteurs d'une pluralité d'éléments des premières informations de base et génère un premier indice inversé dans lequel sont associés une position des premières informations de base dans un fichier du premier espace, une position du vecteur des premières informations structurelles et le vecteur.

10. Procédé de traitement d'informations selon la revendication 9, dans lequel la génération de l'indice inversé inclut la correction des vecteurs d'une pluralité d'éléments de premières informations structurelles similaires, sur la base des vecteurs de la pluralité d'éléments de premières informations structurelles similaires.

11. Procédé de traitement d'informations selon la revendication 8, dans lequel le premier espace est un espace génomique qui utilise une protéine comme premières informations de base, le deuxième espace est un espace chimique qui utilise un groupe fonctionnel comme deuxièmes informations de base et le troisième espace est un espace textuel qui utilise un nom de la protéine ou le groupe fonctionnel comme troisièmes informations de base.

12. Procédé de traitement d'informations selon la revendication 9, le traitement comprenant en outre :
le calcul d'un vecteur d'un récepteur, d'un vecteur d'un ligand et d'un vecteur d'un antagoniste qui appartient aux premières informations structurelles, concernant des données d'enseignement qui utilisent le récepteur comme données d'entrée et l'un parmi le ligand ou l'antagoniste comme étiquette de réponse correcte et génèrent un modèle d'apprentissage, sur la base du vecteur du récepteur,
du vecteur du ligand et du vecteur de l'antagoniste, en réglant une différence entre les données de sortie et l'étiquette de réponse correcte comme fonction de perte, en rétro-propageant une erreur de la fonction de perte et en apprenant un paramètre du modèle d'apprentissage de telle sorte qu'une valeur de la fonction de perte soit minimisée ;
en réponse à la réception d'une demande de recherche, l'entrée du vecteur de la demande de recherche correspondant à un récepteur dans le modèle d'apprentissage pour acquérir un vecteur de données de sortie, la détection d'une séquence de base et d'une formule rationnelle correspondant à un ligand ou à un antagoniste correspondant au vecteur des données de sortie, et la délivrance en sortie de la séquence de base et de la formule rationnelle comme résultats de détection.

13. Dispositif (100, 200) de traitement d'informations comprenant :
une unité de calcul (153) configurée pour calculer des vecteurs d'une pluralité d'éléments d'informations de base spécifiques à un espace définies dans une pluralité d'espaces, la pluralité d'espaces comprenant un espace génomique, un espace chimique et un espace textuel, en mettant en œuvre un plongement de Poincaré sur la pluralité d'éléments d'informations de base, sur la base d'un tableau de concept commun qui classe la pluralité d'éléments d'informations de base avec un concept commun et calcule un vecteur d'informations structurelles avec une granularité plus élevée que les informations de base en ajoutant des vecteurs de la pluralité d'éléments d'informations de base sur lesquelles le plongement de Poincaré a été mis en œuvre et dont les éléments d'informations de base sont inclus dans les informations structurelles ;
une unité de génération (154) configurée pour générer un indice inversé qui définit une relation entre une position des informations de base dans un fichier qui correspond au même espace et le vecteur des informations de base et une relation entre une position des informations structurelles dans le fichier et le vecteur des informations structurelles ; et
une unité de recherche (155) configurée pour, en réponse à la réception d'une demande de recherche, évaluer une similarité entre un vecteur de la demande de recherche et des vecteurs des indices inversés, rechercher un vecteur similaire,
le vecteur similaire étant un vecteur similaire au vecteur de la demande de recherche parmi les vecteurs des indices inversés, acquérir des informations concernant des éléments d'informations de base correspondant au vecteur similaire et délivrer en sortie les informations acquises en tant que résultat de recherche de la demande de recherche.

14. Dispositif (100, 200) de traitement d'informations selon la revendication 13, dans lequel l'unité de calcul (153) calcule un vecteur de chacune de premières informations de base spécifiques pour un premier espace définies dans le premier espace, de deuxièmes informations de base spécifiques pour un deuxième espace définies dans le deuxième espace et de troisièmes informations de base définies dans un troisième espace en mettant en œuvre un plongement de Poincaré sur les premières informations de base, les deuxièmes informations de base et les troisièmes informations de base, sur la base d'un tableau de concept commun qui classe les premières informations de base, les deuxièmes informations de base et les troisièmes informations de base avec un concept commun.

15. Dispositif (100, 200) de traitement d'informations selon la revendication 14, dans lequel l'unité de génération (154) calcule un vecteur de premières informations structurelles avec une granularité plus élevée que les premières informations de base dans le premier espace, sur la base des vecteurs d'une pluralité d'éléments des premières informations de base et génère un premier indice inversé dans lequel sont associés une position des premières informations de base dans un fichier du premier espace, une position du vecteur des premières informations structurelles et le vecteur.

16. Dispositif (100, 200) de traitement d'informations selon la revendication 15, dans lequel l'unité de génération (154) corrige les vecteurs d'une pluralité d'éléments de premières informations structurelles similaires, sur la base des vecteurs de la pluralité d'éléments de premières informations structurelles similaires.

17. Dispositif (100, 200) de traitement d'informations selon la revendication 14, dans lequel le premier espace est un espace génomique qui utilise une protéine comme premières informations de base, le deuxième espace est un espace chimique qui utilise un groupe fonctionnel comme deuxièmes informations de base et le troisième espace est un espace textuel qui utilise un nom de la protéine ou le groupe fonctionnel comme troisièmes informations de base.

18. Dispositif (100, 200) de traitement d'informations selon la revendication 15, comprenant en outre :
une unité d'apprentissage (253) configurée pour calculer un vecteur d'un récepteur, un vecteur d'un ligand et un vecteur d'un antagoniste qui appartient aux premières informations structurelles, concernant des données d'enseignement qui utilisent le récepteur comme données d'entrée et l'un parmi le ligand ou l'antagoniste comme étiquette de réponse correcte et génèrent un modèle d'apprentissage, sur la base du vecteur du récepteur, du vecteur du ligand et du vecteur de l'antagoniste, en réglant une différence entre les données de sortie et l'étiquette de réponse correcte comme fonction de perte, en rétro-propageant une erreur de la fonction de perte et en apprenant un paramètre du modèle d'apprentissage de telle sorte qu'une valeur de la fonction de perte soit minimisée ;
une unité d'exécution (254) configurée pour, en réponse à la réception d'une demande de recherche, entrer le vecteur de la demande de recherche correspondant à un récepteur dans le modèle d'apprentissage pour acquérir un vecteur de données de sortie, détecter une séquence de base et une formule rationnelle correspondant à un ligand ou à un antagoniste correspondant au vecteur des données de sortie, et délivrer en sortie la séquence de base et la formule rationnelle comme résultats de détection.
